(19) Europäisches Patentamt
European Patent Office
Office européen des brevets

(11) **EP 4 337 310 B1**

(12) **EUROPÄISCHE PATENTSCHRIFT**

(45) Veröffentlichungstag und Bekanntmachung des Hinweises auf die Patenterteilung:
**16.04.2025   Patentblatt 2025/16**

(21) Anmeldenummer: **22724418.3**

(22) Anmeldetag: **10.05.2022**

(51) Internationale Patentklassifikation (IPC):
*A61N 5/06* (2006.01)     *A61B 1/06* (2006.01)
*A61B 1/00* (2006.01)     *A61B 1/273* (2006.01)
*A61B 5/00* (2006.01)

(52) Gemeinsame Patentklassifikation (CPC):
**A61N 5/062; A61N 5/0601;** A61B 1/00135;
A61B 1/06; A61B 1/2733; A61B 2018/2005;
A61N 2005/0609; A61N 2005/0612;
A61N 2005/0651

(86) Internationale Anmeldenummer:
**PCT/DE2022/200097**

(87) Internationale Veröffentlichungsnummer:
**WO 2022/237943 (17.11.2022 Gazette 2022/46)**

(54) **LICHTAPPLIKATORSYSTEM MIT SCHUTZHÜLSE**

LIGHT APPLICATOR SYSTEM WITH PROTECTIVE SLEEVE

SYSTÈME APPLICATEUR DE LUMIÈRE AVEC MANCHON DE PROTECTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **14.05.2021   DE 102021204908**

(43) Veröffentlichungstag der Anmeldung:
**20.03.2024   Patentblatt 2024/12**

(73) Patentinhaber: **Richard Wolf GmbH
75438 Knittlingen (DE)**

(72) Erfinder:
• **WEBER, Bernd Claus
  76228 Karlsruhe (DE)**
• **SIEBER, Stephan
  75438 Knittlingen-Freudenstein (DE)**

(74) Vertreter: **Patentanwälte Hemmer Lindfeld Frese Partnerschaft mbB
Wallstraße 33a
23560 Lübeck (DE)**

(56) Entgegenhaltungen:
DE-A1- 102019 129 556     DE-A1- 102019 212 197
DE-T2- 68 929 404         US-A- 6 048 359
US-A1- 2021 016 102

**Beschreibung**

Die vorliegende Offenbarung betrifft ein Lichtapplikatorsystem zur Untersuchung und/oder Behandlung eines organischen Körpers, insbesondere zur photodynamischen Therapie (PDT) von pathologischem Gewebe.

Es ist bekannt, Endoskope dazu zu nutzen, um Videoaufnahmen vom Inneren eines menschlichen oder tierischen Körpers zu Zwecken der medizinischen Diagnose und/oder Therapie zu machen. Dabei ist es ein ständiges Bestreben, den Einführabschnitt von Endoskopen möglichst dünn auszugestalten, damit möglichst kleine Hohlräume eingesehen werden können und das Gewebe dabei möglichst wenig in Mitleidenschaft zu ziehen.

Endoskope werden allerdings nicht nur dazu genutzt, um Bild- oder Videoaufnahmen zu machen, sondern auch als Diagnose- oder Therapiemittel selbst eingesetzt. Beispielsweise für die Detektion und Lokalisierung von prä- und frühmalignem Gewebe kann eine Fluoreszenz-Endoskopie eingesetzt werden, bei der es nicht auf eine natürliche Echtfarb-Darstellung des Gewebes ankommt, sondern lediglich auf eine Fluoreszenzanregung, mit der sich pathologisches Gewebe von gesundem Gewebe unterscheiden lässt. Dabei kann das mittels Lichtstrahlung angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise im Rahmen einer photodynamischen Diagnose (PDD) und/oder photodynamischen Therapie (PDT) mittels eines Photosensibilisators bzw. Markerstoffs durchgeführt werden, der sich selektiv an pathologischem Gewebe anreichert.

Bei der photodynamischen Therapie (PDT) wird Licht mittels eines Lichtapplikators unmittelbar auf oder sogar in pathologisches Gewebe appliziert, um lichtinduziert die Bildung von Sauerstoffradikalen mittels des örtlich begrenzt angereicherten Photosensibilisators bzw. Markerstoffs zu fördern und dadurch das pathologische Gewebe, wie etwa einen Tumor, zu zerstören. Typischerweise wird dazu Laserlicht in einen Lichtleiter eingekoppelt und zum Gewebe geleitet. Wenn das pathologische Gewebe flächig auf einer äußeren Oberfläche, z. B. der Haut, oder einer inneren Oberfläche, z. B. der Speiseröhreninnenfläche oder Darmwandung, angeordnet ist, dann kann das Therapielicht relativ einfach ausgekoppelt und auf die pathologische Gewebefläche gestrahlt werden. Erstreckt sich allerdings das pathologische Gewebe über ein Volumen, so kann wegen der begrenzten Eindringtiefe des Lichts in das Gewebe nicht immer ein Tumor von "außen" effektiv bestrahlt werden. In diesem Fall ist die PDT besonders effektiv, wenn das Licht vom Inneren des pathologischen Gewebevolumens aus möglichst isotrop abgestrahlt wird. Dazu muss der Lichtapplikator in das pathologische Gewebe eingestochen werden. Dies wird auch als interstitielle (durch innere Oberflächen) und/oder perkutane (durch die Haut) PDT bezeichnet.

In der US 6,048,359 ist beispielsweise beschrieben, wie mehrere Lichtapplikatoren mit Hilfe eines Positionierungsrasters in pathologisches Gewebe eingestochen werden. Die DE 10 2019 129 556 A1 beschreibt einen Lichtapplikator mit einer starren Einführhülse, um ein flexibles Leiterelement für den Einstich zu stabilisieren. Die DE 10 2019 212 197 A1 beschreibt ein Lichtapplikatorsystem mit mindestens einem Lichtapplikator, der beim Anschluss an eine Betriebseinheit identifizierbar ist.

Nachteilig an den bekannten Lösungen ist, dass die sehr scharfe Applikatorspitze zum einen gefährlich scharf für einen Anwender ist und zum anderen bei der Handhabung vor dem eigentlichen Einstich in die Haut des Patienten leicht abbrechen kann.

Daraus ergibt sich die Aufgabe, einen Lichtapplikator bereitzustellen, bei dem die scharfe und empfindliche Applikatorspitze bis zum eigentlichen Einstich derart geschützt ist, dass sie den Anwender nicht gefährdet und vor dem eigentlichen Einstich in die Haut des Patienten nicht abbricht.

Gemäß der vorliegenden Offenbarung wird zur Lösung dieses Problems ein Lichtapplikatorsystem zur Untersuchung und/oder Behandlung von einem organischen Körper bereitgestellt, wobei das Lichtapplikatorsystem zumindest einen Lichtapplikator und ein Positionierungselement aufweist, wobei der Lichtapplikator einen distalseitigen Einführabschnitt mit zumindest einem aktiv lichtemittierenden Element, z.B. einer LED am distalen Ende zum Einstechen in Gewebe des organischen Körpers aufweist, wobei der Einführabschnitt eine sich zumindest teilweise distal von dem zumindest einen aktiv lichtemittierenden Element angeordnete und sich distalwärts spitz verjüngende Nadelspitze aufweist, wobei die Nadelspitze als lichttransparenter Streukörper zur Streuung von in distale Richtung abgegebenem Licht ausgeführt sein kann, wobei das Positionierungselement zumindest temporär in einer definierten Position und Ausrichtung zum organischen Körper fixierbar ist und zumindest eine Aufnahme für den mindestens einen Lichtapplikator aufweist, in welcher der mindestens eine Lichtapplikator zumindest temporär eine definierte Ausrichtung zum organischen Körper aufweist, wobei der Lichtapplikator eine relativ zum Einführabschnitt axial bewegliche Schutzhülse aufweist, welche in einer ersten Axialposition relativ zum Einführabschnitt die Nadelspitze schützend umschließt und in einer durch eine Axialposition des Einführabschnitts (1) relativ zum Positionierungselement bestimmten zweiten Axialposition relativ zum Einführabschnitt proximalwärts von der Nadelspitze zurückgeschoben ist, wobei die Schutzhülse als Einführhülse zum sicheren Einführen in die mindestens eine Aufnahme des Positionierungselements dient.

Bei dem hier offenbarten Lichtapplikator wird also kein Laserlichtleiter eingesetzt, sondern das Diagnose- oder Therapielicht in situ am distalen Ende des Lichtapplikators durch ein aktiv lichtemittierendes Element, z.B. eine miniaturisierte LED erzeugt, z.B. mit einer lateralen Breite von weniger als 1 mm, wobei "aktiv" bedeutet, dass das lichtemittierende Element elektrische Energie aufnimmt und in Licht umwandelt, also nicht nur in Form eines Lichtleiters weiterleitet. Ein teurer Laser wird daher nicht benötigt, sodass die Kosten stark reduziert sind. Der hierin offenbarte Lichtapplikator, oder zumindest dessen Einführabschnitt, kann sehr günstig hergestellt werden und somit als steriler Einweg-Artikel zum einmaligen Gebrauch realisiert werden, was aufwendige Reinigungen und Sterilisationen beim Anwender obsolet macht. Bei größeren Tumoren oder ganzen pathologischen Organen oder Organbereichen kann das Lichtapplikatorsystem eine Mehrzahl an Lichtapplikatoren aufweisen, die gleichzeitig für die PDT eingesetzt werden, indem sie verteilt über das gesamte Organ eingestochen werden, um das gesamte Organ homogen auszuleuchten. Da sich der Photosensibilisator bzw. Markerstoff selektiv nur in pathologischem Gewebe anreichert und dort unter dem Lichteinfluss reagiert, wird gesundes Gewebe durch das Licht nicht beschädigt. Zum einen muss das pathologische Gewebe dann zuvor nicht mehr so genau lokalisiert werden und zum anderen wird das Risiko verringert, dass pathologisches Gewebe unbemerkt untherapiert bleibt.

Für die perkutane PDT mit mehreren Lichtapplikatoren kann es sinnvoll sein, dass das Positionierungselement eine Vielzahl von Aufnahmen aufweist und eine an oder auf die Haut des Patienten definiert platzierbare und/oder klebbare organspezifische Schablone bildet, um einem Anwender damit Einstichstellen, -winkel und/oder -tiefen für die Lichtapplikatoren anzuzeigen und eine möglichst vollständige und weitgehend homogene Ausleuchtung des Organs zu erzielen.

Der Lichtapplikator kann allerdings nicht nur für die Therapie eingesetzt werden, sondern auch für die Untersuchung, also die Diagnose. Insbesondere im Zusammenspiel mit einem Endoskop oder mit dem Lichtapplikator als Teil eines Endoskops kann die durch den Lichtapplikator erzeugte Fluoreszenz eines in pathologischem Gewebe angereicherten Photosensibilisators bzw. Markerstoffs beobachtet werden.

Optional kann die Schutzhülse zumindest abschnittsweise einen Außendurchmesser aufweisen, der passgenau in einen Innendurchmesser der mindestens einen Aufnahme des Positionierungselements passt. Damit wird nur eine exakt koaxiale Ausrichtung der Schutzhülse in der mindestens einen Aufnahme des Positionierungselements erlaubt und es werden etwaige Verschwenkungen verhindert. Sobald sich die Schutzhülse in der mindestens einen Aufnahme des Positionierungselements befindet, ist nur noch eine axiale Bewegung des Lichtapplikators möglich. Die Schutzhülse wird vorzugsweise distalwärts in die mindestens eine Aufnahme des Positionierungselements geschoben bis die Schutzhülse eine axiale Sollposition erreicht, welche vorzugsweise eine maximale Distalposition der Schutzhülse ist. Beispielsweise kann die Schutzhülse beim distalwärtigen Einschieben in die mindestens eine Aufnahme des Positionierungselements bei Erreichen der Sollposition mit einem Anschlag gegen das Positionierungselement anschlagen.

Optional kann sich der Außendurchmesser der Schutzhülse am distalen Ende distalwärts verjüngen und/oder es kann sich der Innendurchmesser der mindestens einen Aufnahme des Positionierungselements am proximalen Ende proximalwärts aufweiten. Damit wird das Einführen der Schutzhülse in die Aufnahme des Positionierungselements erleichtert.

Optional kann der Einführabschnitt des Lichtapplikators starr sein und in Axialrichtung eine größere Länge aufweisen als die Schutzhülse. Der Einführabschnitt entspricht vorzugsweise einer möglichst dünnen starren Nadel, an deren lichtstreuender Spitze das Licht einer in oder an der Spitze angeordneten miniaturisierten LED angeordnet ist.

Optional kann die Schutzhülse unverlierbar am Einführabschnitt des Lichtapplikators gesichert sein. Damit schützt die Schutzhülse die Nadelspitze auch außerhalb der eigentlichen Anwendung, beispielsweise beim Transport und/oder bei der Entsorgung des Lichtapplikators.

Optional kann der Lichtapplikator proximalseitig ein Handgriffelement zur manuellen Positionierung des Lichtapplikators aufweisen. Solange die Ausrichtung durch die eingeführte Schutzhülse im Positionierungselement noch nicht festgelegt ist, kann das Handgriffelement auch zur Ausrichtung des Lichtapplikators außerhalb des Positionierungselements dienen. Das Handgriffelement kann fest mit dem Einführabschnitt verbunden sein oder mit diesem lösbar koppelbar sein. Das Handgriffelement kann ggf. mehrfach verwendbar sein und der abkoppelbare Einführabschnitt als Einweg-Artikel zum einmaligen Gebrauch vorgesehen sein.

Optional kann die Schutzhülse bei einem distalwärtigen Einführen des Lichtapplikators in die mindestens eine Aufnahme des Positionierungselements so lange in der ersten Axialposition gesichert sein bis die Schutzhülse in der mindestens einen Aufnahme des Positionierungselements eine Sollposition im Positionierungselement erreicht, bei welcher der Einführabschnitt distalwärts aus der Schutzhülse schiebbar ist. Vorzugsweise ist die Sollposition eine maximale Distalposition der Schutzhülse in der Aufnahme des Positionierungselements. In der Sollposition befindet sich vorzugsweise die Schutzhülse vollständig oder zumindest zum Großteil innerhalb der Aufnahme des Positionierungselements und ist darin eingerastet.

Optional kann die Schutzhülse bei einem proximalwärtigen Herausziehen des Lichtapplikators aus der mindestens einen Aufnahme des Positionierungselements so lange in der Sollposition im Positionierungselement gesichert sein bis die Schutzhülse die erste Axialposition bezüglich des Einführabschnitts einnimmt, bei welcher die Schutzhülse proximalwärts aus der mindestens einen Aufnahme des Positionierungselements herausziehbar ist.

Optional kann das Lichtapplikatorsystem ferner ein elastisch verformbares und/oder bewegbares Eingriffselement aufweisen, wobei das Eingriffselement derart zwischen dem Einführabschnitt und der Schutzhülse angeordnet ist, dass es bei Überwindung einer Axialkraft so weit elastisch nachgibt oder sich bewegt, dass die Schutzhülse die erste Axialposition distalwärts erreichen und/oder proximalwärts verlassen kann. Optional kann das erste Eingriffselement Teil der Schutzhülse und/oder des Einführabschnitts sein.

Optional kann das Lichtapplikatorsystem ein weiteres elastisch verformbares und/oder bewegbares Eingriffselement aufweisen, wobei das zweite Eingriffselement derart zwischen der Schutzhülse und der mindestens einen Aufnahme des Positionierungselements angeordnet ist, dass es bei Überwindung der zweiten Axialkraft so weit elastisch nachgibt oder sich bewegt, dass die Schutzhülse die Sollposition im Positionierungselement distalwärts erreichen und/oder proximalwärts verlassen kann. Optional kann die das zweite Eingriffselement Teil der Schutzhülse oder des Positionierungselements sein.

Optional kann distalwärts die erste Axialkraft größer sein als die zweite Axialkraft und proximalwärts die zweite Axialkraft größer sein als die erste Axialkraft.

Die Begriffe "distalwärtig" bzw. "proximalwärtig" sollen hierin eine relative Position bedeuten, die sich distal bzw. proximal von einem Anwender des Systems als Bezugsposition befindet. Die Begriffe "distalseitig" bzw. "proximalseitig" sollen hierin entsprechend Positionen an einer distalen bzw. proximalen Seite eines Gegenstands bedeuten. Die Begriffe "distalwärts" bzw. "proximalwärts" sollen hierin entsprechend Richtungen bedeuten, die sich nach distal bzw. proximal erstrecken.

Die Offenbarung ist nachfolgend anhand von in den Zeichnungen dargestellten Ausführungsbeispielen näher erläutert. Es zeigen:

Fig. 1a,b zwei schematische Längsschnittdarstellungen von Ausführungsbeispielen einer Nadelspitze eines Einführabschnitts eines hierin offenbarten Lichtapplikatorsystems;

Fig. 2 eine schematische Längsschnittdarstellung des in Fig. 1a gezeigten Ausführungsbeispiels einer Nadelspitze eines Einführabschnitts bei seitlicher Krafteinwirkung;

Fig. 3a,b zwei schematische Längsschnittdarstellungen zur Erläuterung des Zustandekommens einer seitlichen Krafteinwirkung bei der Anwendung eines hierin offenbarten Lichtapplikatorsystems;

Fig. 4 eine schematische Längsschnittdarstellung eines Ausführungsbeispiels eines hierin offenbarten Lichtapplikatorsystems vor einem Einführen des Lichtapplikators in das Positionierungselement;

Fig. 5 eine schematische Längsschnittdarstellung eines Ausführungsbeispiels eines hierin offenbarten Lichtapplikatorsystems bei einem Verkanten während des Einführens des Lichtapplikators in das Positionierungselement;

Fig. 6 eine schematische Längsschnittdarstellung eines Ausführungsbeispiels eines hierin offenbarten Lichtapplikatorsystems beim Einführen des Lichtapplikators in das Positionierungselement mit der Schutzhülse in der ersten Axialposition bezüglich des Einführabschnitts;

Fig. 7 eine schematische Längsschnittdarstellung eines Ausführungsbeispiels eines hierin offenbarten Lichtapplikatorsystems beim Einstechen des Einführabschnitts in den Körper;

Fig. 8 eine schematische Längsschnittdarstellung eines Ausführungsbeispiels eines hierin offenbarten Lichtapplikatorsystems beim Durchstechen des Einführabschnitts durch den Körper und beim Einstechen in das zu untersuchende/behandelnde Gewebe;

Fig. 9 eine schematische Längsschnittdarstellung eines Ausführungsbeispiels des Lichtapplikators eines hierin offenbarten Lichtapplikatorsystems;

Fig. 10 eine schematische Längsschnittdarstellung eines Ausführungsbeispiels der Schutzhülse im Positionierungselement eines hierin offenbarten Lichtapplikatorsystems, wobei der Lichtapplikator nicht gezeigt ist;

Fig. 11 eine schematische Längsschnittdarstellung eines Ausführungsbeispiels eines hierin offenbarten Lichtapplikatorsystems beim Einstechen des Einführabschnitts in den Körper, wobei die Schutzhülse nicht in der ersten Axialposition bezüglich des Einführabschnitts sitzt;

Fig. 12 eine schematische Längsschnittdarstellung eines Ausführungsbeispiels des Lichtapplikators beim Einführen in das Positionierungselement mit der Schutzhülse in der ersten Axialposition bezüglich des Einführabschnitts und bevor die Schutzhülse eine axiale Sollposition im Positionierungselement erreicht hat;

Fig. 13 eine schematische Längsschnittdarstellung eines Ausführungsbeispiels des Lichtapplikators beim Einführen in das Positionierungselement mit der Schutzhülse in der ersten Axialposition bezüglich des Einführabschnitts, wobei die Schutzhülse gerade die axiale Sollposition im Positionierungselement erreicht hat;

Fig. 14 eine schematische Längsschnittdarstellung eines Ausführungsbeispiels des Lichtapplikators beim distalwärtigen Herausdrücken der Nadelspitze des Einführabschnitts aus der sich in der axialen Sollposition im Positionierungselement befindenden Schutzhülse in den Körper;

Fig. 15 eine schematische Längsschnittdarstellung eines Ausführungsbeispiels des Lichtapplikators beim proximalwärtigen Hineinziehen der Nadelspitze des Einführabschnitts aus dem Körper in die sich in der axialen Sollposition im Positionierungselement befindenden Schutzhülse;

Fig. 16 eine schematische Längsschnittdarstellung eines Ausführungsbeispiels des Lichtapplikators beim Herausziehen aus dem Positionierungselement, wobei die Schutzhülse bezüglich des Einführabschnitts gerade die erste Axialposition eingenommen hat, aber sich bezüglich des Positionierungselements noch in der axialen Sollposition befindet;

Fig. 17 eine schematische Längsschnittdarstellung eines Ausführungsbeispiels des Lichtapplikators beim Herausziehen aus dem Positionierungselement mit der Schutzhülse in der ersten Axialposition bezüglich des Einführabschnitts und nachdem die Schutzhülse die axiale Sollposition im Positionierungselement proximalwärts verlassen hat;

Fig. 18 eine schematische Längsschnittdarstellung eines Ausführungsbeispiels eines hierin offenbarten Lichtapplikatorsystems zur Erläuterung der Fixierung des Positionierungselements relativ zum Körper des Patienten;

Fig. 19 bis 26 schematische Längsschnittdarstellungen mit Detailausschnitten eines Ausführungsbeispiels eines hierin offenbarten Lichtapplikatorsystems analog zu Fig. 11 bis 17 in verschiedenen Positionen des Lichtapplikators; und

Fig. 27 eine schematische Längsschnittdarstellung eines alternativen Ausführungsbeispiels eines hierin offenbarten Lichtapplikatorsystems.

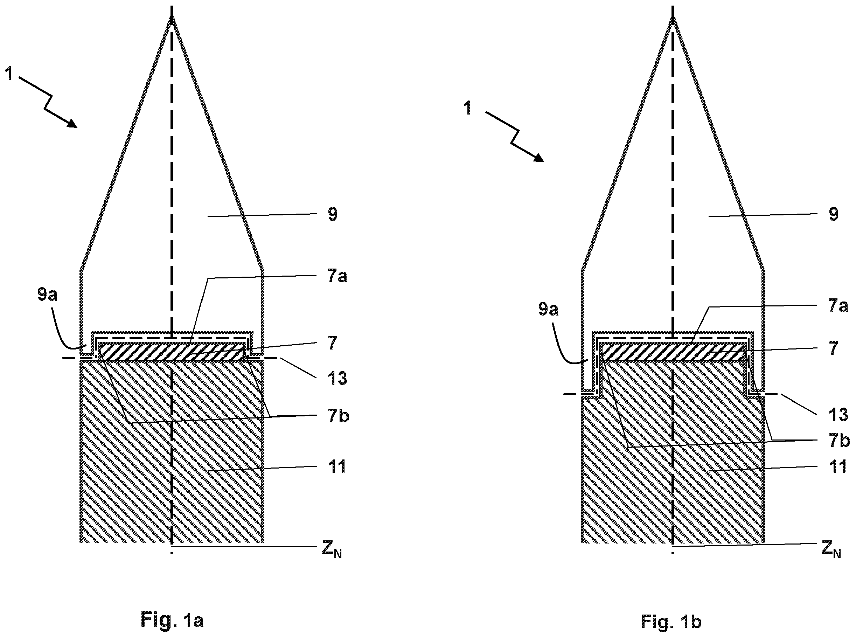

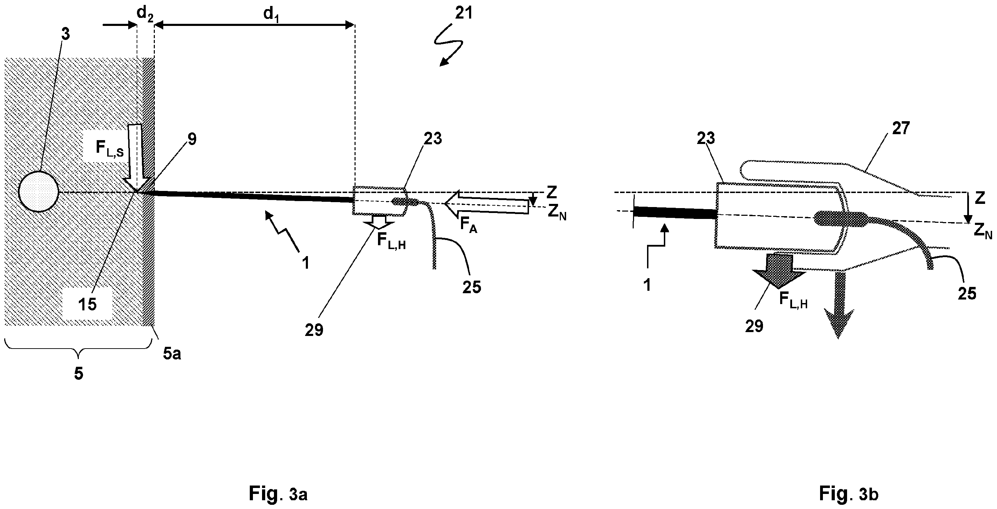

Fig. 1a, 1b, 2, 3a und 3b verdeutlichen das mit dem hierin offenbarten Lichtapplikatorsystem gelöste Problem. Fig. 1a und 1b zeigen verschiedene Ausführungsformen eines distalen Endes eines Einführabschnitts 1 eines Lichtapplikators eines hierein offenbarten Lichtapplikatorsystems. Das distale Ende dient dem Einstechen in Gewebe 3 eines organischen Körpers 5 (siehe Fig. 3a und 3b), um das Gewebe 3 zur photodynamischen Therapie (PDT) oder Diagnose (PDD) mit Licht zu bestrahlen. Der Einführabschnitt 1 des Lichtapplikators ist für eine möglichst minimalinvasive Behandlung oder Diagnose nadelförmig, möglichst dünn (mit einem Durchmesser von beispielsweise weniger als 2 mm) und starr ausgebildet.

Das distale Ende des Einführabschnitts 1 weist hier beispielhaft ein aktiv lichtemittierendes Element 7 in Form einer LED auf, deren Hauptabstrahlrichtung in Längsrichtung $Z_N$ des Einführabschnitts 1 gerichtet ist. Zumindest teilweise distal von der LED 7 ist eine sich distalwärts spitz verjüngende Nadelspitze 9 angeordnet, die einen lichttransparenten Streukörper zur Streuung des Lichts der LED 7 aufweist. Die Nadelspitze 9 ist hier im Wesentlichen einstückig aus dem lichttransparenten Streukörper gebildet, der beispielweise Kunststoff mit einem oder mehr Verstärkungselementen aufweisen kann.

Die LED 7 ist an einer distalen Stirnseite eines Leiterelements 11 angeordnet. Das Leiterelement 11 ist hier als massiver Metallstab ausgebildet. Das Leiterelement 11 kann sowohl als elektrischer Leiter zur Stromversorgung der LED 7 dienen als auch als Wärmeleiter zum proximalwärtigen Ableiten von Wärme, die durch die LED 7 erzeugt wird. Dazu kann ein Kern des Leiterelements 11 beispielsweise Kupfer aufweisen, das gut wärme- und stromleitend ist. Zur Versteifung des Leiterelements 11, das einen Durchmesser von 1 mm oder weniger haben kann, kann ein Mantel des Leiterelements 11 aus einem biegesteiferen Material, wie etwa Stahl, ausgebildet sein. Wenn Kern und Mantel des Leiterelements 11 gegeneinander elektrisch isoliert sind, beispielsweise durch eine dünne Isolationsschicht zwischen ihnen, können Kern und Mantel als Hin- und Rückleiterpaar zur Stromversorgung der LED 7 fungieren.

Wie in Figuren 1a und 1b gezeigt, ist die Querschnittsfläche des Leiterelements 11 nur wenig größer als die Querschnittsfläche der LED 7. Um die Nadelspitze 9 einerseits nicht ausschließlich an einer Stirnfläche 7a der LED 7 zu befestigen und andererseits mit der Nadelspitze 9 nicht über die Querschnittsfläche des Leiterelements 11 hinauszugehen, weist die Nadelspitze 9 einen proximalseitigen hülsenförmigen Abschnitt 9a auf, der die LED 7 umfangseitig umgreift und die Nadelspitze 9 direkt mit der Stirnseite des Leiterelements 11 verbindet. Über eine flächige Verbindungsstelle 13 ist die Nadelspitze 9 mit der LED 7 und dem Leiterelement 11 verbunden, vorzugsweise stoffschlüssig über eine Verklebung entlang der Verbindungsstelle 13. Zur Vergrößerung der flächigen Verbindungsstelle 13 direkt zwischen Nadelspitze 9 und Leiterelement 11 ist in der in Fig. 1b gezeigten Ausführungsform der proximalseitige hülsenförmige Abschnitt 9a axial länger ausgeführt und das Leiterelement 11 weist einen entsprechend verjüngten distalen Abschnitt auf, der von dem hülsenförmigen Abschnitt 9a der Nadelspitze 9 umfangseitig umgriffen wird. Dadurch kann die Nadelspitze 9 nicht nur stirnseitig direkt mit dem Leiterelement 11 verklebt werden, sondern auch umfangseitig am verjüngten distalen Abschnitt des Leiterelements 11. Damit wird die Verbindungsstelle 13 größer und verstärkt.

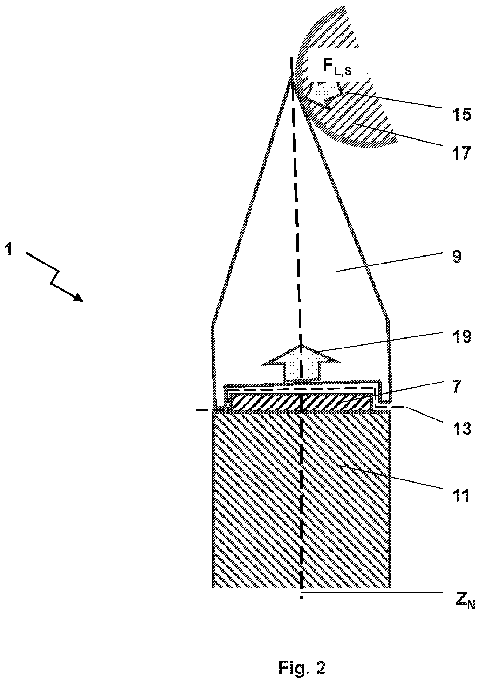

Fig. 2 zeigt nämlich, dass die Nadelspitze 9 leicht abreißen oder abbrechen kann. Da die Nadelspitze 9 für eine minimalinvasive Behandlung oder Therapie möglichst spitz sein muss, ist sie vorzugsweise in Längsrichtung $Z_N$ wesentlich länger als ihr Durchmesser. Wird nun eine seitliche Kraft $F_{L,S}$ 15 durch einen externen Körper 17 auf das distale Nadelspitzenende der Nadelspitze 9 ausgeübt, so wirkt durch Hebelwirkung eine vergrößerte Ablösekraft 19 an der Verbindungsstelle 13, die daraufhin wie gezeigt reißen kann. Diese Gefahr besteht auch für die Ausführungsform gemäß Fig. 1b, wenngleich die Verbindungsstelle 13 dort prinzipiell stärker ist. Allerdings besteht in beiden Ausführungsformen gemäß Fig. 1a und 1b die Gefahr, dass der proximalseitige hülsenförmige Abschnitt 9a vom Rest der Nadelspitze 9 abbrechen kann, da der hülsenförmige Abschnitt 9a nur sehr dünn ausgestaltet werden kann.

Die Fig. 1a, 1b und 2 zeigen beispielhaft eine Ausführungsform, bei der es sich bei dem aktiv lichtemittierenden Element um eine einzelne LED 7 handelt, die an der distalen Stirnseite des Leiterelements 11 angeordnet ist. Alternativ zu der einen LED 7 können auch mehrere aktiv lichtemittierende Elemente zum Einsatz kommen und es kann sich dabei auch um andere aktiv lichtemittierende Elemente, z.B. Laserdioden, handeln. Alternativ zur Platzierung an der distalen Stirnseite des Leiterelements 11 kann das mindestens eine aktiv lichtemittierende Element auch an der Mantelfläche des distalen Einführabschnitts 1 bzw. des Leiterelements 11 des Lichtapplikators 21 platziert sein. Außerdem muss es sich bei der Nadelspitze 9 nicht unbedingt um ein separates Teil handeln, welches mit dem distalen Ende des Einführabschnitts 1 verklebt ist. Stattdessen kann die Nadelspitze 9 auch eine Fortführung des Leiterelements 11 sein. Dann besteht die wesentliche Aufgabe darin, den Anwender und den Patienten vor einer unbeabsichtigten Berührung mit der spitzen Nadelspitze 9 und einer daraus möglicherweise resultierenden Verletzung zu schützen. In den nachfolgenden Ausführungen wird beispielhaft die an der distalen Stirnseite des Leiterelements 11 angeordnete LED 7 mit der distalseitig aufgebrachten, beispielsweise verklebten Nadelspitze 9, die als Streukörper ausgeführt ist, betrachtet.

Fig. 3a und 3b zeigen, wie es bei Anwendung eines hierin offenbarten Lichtapplikatorsystems 21 zur Einwirkung einer seitlichen Krafteinwirkung $F_{L,S}$ 15 auf die Nadelspitze 9 kommen kann. Das Lichtapplikatorsystem weist einen Lichtapplikator 21 auf, der den Einführabschnitt 1, ein Handgriffelement 23 und eine über ein Anschlusskabel 25 anschließbare Stromversorgungseinheit (nicht gezeigt) umfasst.

Eine Bedienperson greift mit ihrer Hand 27 das Handgriffelement 23, um den Einführabschnitt 1 in Richtung des zu untersuchenden oder behandelnden Gewebes 3 durch die Haut 5a in den Körper 5 des Patienten zu stechen. In Fig. 3a und 3b sind schematisch die Größenverhältnisse angedeutet, die sich hier nicht maßstabsgetreu darstellen lassen. Dabei hat der Einführabschnitt 1 eine relativ große Länge $d_1 + d_2$ vom distalen Ende der Nadelspitze 9 bis zum Handgriffelement 23, z.B. 20 cm oder mehr, und ist sehr dünn, z.B. mit einem Durchmesser von 1 mm oder weniger. Die Länge der Nadelspitze 9 ist hier mit $d_2$ bezeichnet und der Rest der Länge des Einführabschnitts 1 mit $d_1$, wobei $d_1 \gg d_2$. Durch diese Größenverhältnisse ergibt sich aus dem Hebelprinzip eine relativ große seitlich auf die Nadelspitze 9 einwirkende Kraft $F_{L,S}$ 15, wenn die Bedienperson nur eine relativ kleine seitliche Kraft $F_{L,H}$ 29 auf das Handgriffelement 23 ausübt. In dem gezeigten Fall wurde zunächst senkrecht in Z-Richtung in die Haut 5a eingestochen, dann wurde aber der Lichtapplikator 21 durch eine bei manueller Handhabung unvermeidbare relativ kleine seitliche manuelle Kraft $F_{L,H}$ 29 auf das Handgriffelement 23 leicht abgewinkelt. Auf die Nadelspitze 9 wirkt dann eine vielfach größere seitliche Kraft $F_{L,S}$ 15, die zum Abriss oder Abbruch der Nadelspitze 9 führen kann, da

$$F_{L,S} \approx \frac{d_1}{d_2} \cdot F_{L,H}$$

gilt.

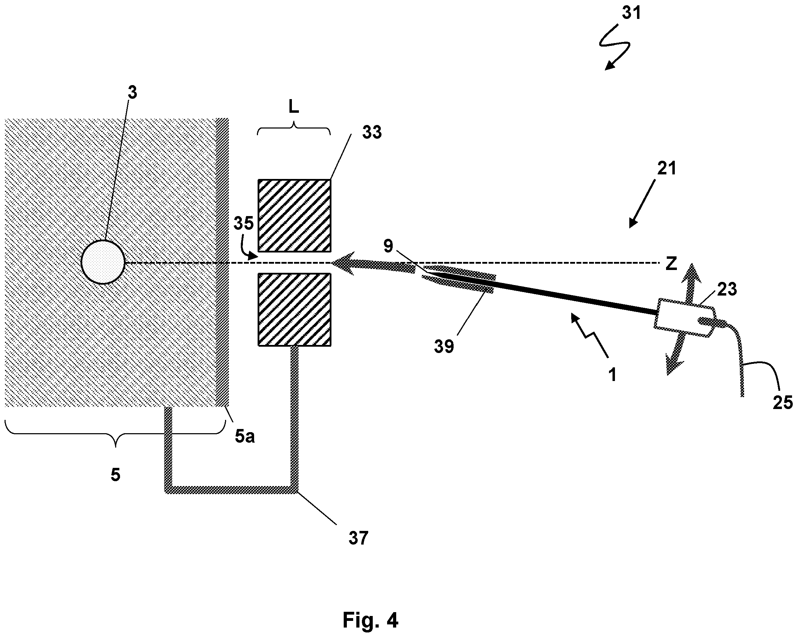

Fig. 4 zeigt ein Lichtapplikatorsystem 31, bei dem das Risiko des Abrisses oder Abbruchs der Nadelspitze 9 erheblich reduziert ist. Das Lichtapplikatorsystem 31 weist einen Lichtapplikator 21 mit einem Einführabschnitt 1, einer Nadelspitze 9 am distalen Ende und einem Handgriffelement 23 am proximalen Ende auf. Zudem weist das Lichtapplikatorsystem 31 ein Positionierungselement 33 auf, das zumindest temporär in eine definierte Position und Ausrichtung relativ zum organischen Körper 5 fixierbar ist. Das Positionierungselement 33 kann, wie in Fig. 4 schematisch gezeigt, einen Abstand zur Haut 5a haben oder direkt in Kontakt mit der Haut 5a angeordnet sein, beispielsweise auf die Haut 5a aufgeklebt sein. Das Positionierungselement 33 kann eine Schablone sein mit mindestens einer Aufnahme 35 für den Lichtapplikator 21. Die Aufnahme 35 kann eine Ausnehmung oder ein Loch im Positionierungselement 33 sein, das die Einstichstelle auf der Haut 5a in einer zur senkrechten Einstichrichtung Z orthogonalen Ebene definiert. In dieser zur senkrechten Einstichrichtung Z orthogonalen Ebene sind vorzugsweise mehrere Aufnahmen 35 angeordnet, um der Bedienperson eine Auswahl aus einer Vorgabe von definierten Einstichstellen zu geben und/oder mehrere Lichtapplikatoren 21 gleichzeitig einzusetzen. Das Positionierungselement 33 und der Körper 5 des Patienten sind vorzugsweise mittels eines Fixierelements 37 relativ zueinander, vorzugsweise beide bezüglich eines festen örtlichen Referenzkörpers, beispielweise eines Behandlungstisches, fixiert oder fixierbar.

In axialer Einstichrichtung Z weist das Positionierungselement 33 eine gewisse Dicke L auf, sodass die Aufnahme 35 in axialer Richtung Z eine Länge L hat. Durch diese Länge L definiert das Positionierungselement 33 nicht nur die Position der Einstichstelle, sondern auch die Ausrichtung des Lichtapplikators 21 bezüglich des Körpers 5. In dem in Fig. 4 gezeigten Fall ist durch die Aufnahme 35 nur ein senkrechter Einstich in die Haut 5a erlaubt. Das Positionierungselement 33 ist dazu vorzugsweise starr und unflexibel ausgebildet.

Da das Risiko des Abrisses oder Abbruches der Nadelspitze 9 beim Verkanten (siehe Fig. 4) während des manuellen Einführens des Einführabschnitts 1 in das starre und unflexible Positionierungselement 33 noch größer ist als bei dem in Fig. 3a und 3b gezeigten verkanteten Einführen direkt in die Haut 5a ohne Positionierungselement 33, weist das Lichtapplikatorsystem 31 eine Schutzhülse 39 auf. Die Schutzhülse 39 ist relativ zum Einführabschnitt 1 axial beweglich, aber in mindestens einer ersten Axialposition relativ zum Einführabschnitt 1 temporär fixiert. In der in Fig. 4 gezeigten ersten Axialposition relativ zum Einführabschnitt 1 umschließt die Schutzhülse 39 die Nadelspitze 9 schützend. Die Schutzhülse 39 ragt in der gezeigten ersten Axialposition axial über das distale Ende der Nadelspitze 9 hinaus. Dadurch ist nicht nur die Nadelspitze 9 geschützt, sondern auch ein Patient und/oder eine Bedienperson vor versehentlichem Stechen mit der sehr spitzen Nadelspitze 9. Die Schutzhülse 39 ist hier derart unverlierbar am Einführabschnitt 1 des Lichtapplikators 21 gesichert, dass sie nicht distalwärts vom Einführabschnitt 1 abfallen oder abgezogen werden kann. Die in Fig. 4 gezeigte erste Axialposition ist die maximal distale Axialposition relativ zum Einführabschnitt 1 des Lichtapplikators 21. In proximale Richtung kann das Handgriffelement 23 oder ein anderer Anschlag (hier nicht gezeigt) die maximale axiale Bewegungsfreiheit der Schutzhülse 39 entlang des Einführabschnitts 1 begrenzen.

Ein Innendurchmesser der Aufnahme 35 ist passgenau auf einen Außendurchmesser der Schutzhülse 39 angepasst, sodass diese passgenau in axialer Richtung Z in die Aufnahme 35 des Positionierungselements 33 eingeschoben werden kann. Um das Einführen in die Aufnahme 35 zu erleichtern, ist der Außendurchmesser der Schutzhülse 39 am distalen Ende der Schutzhülse 39 distalwärts verjüngt.

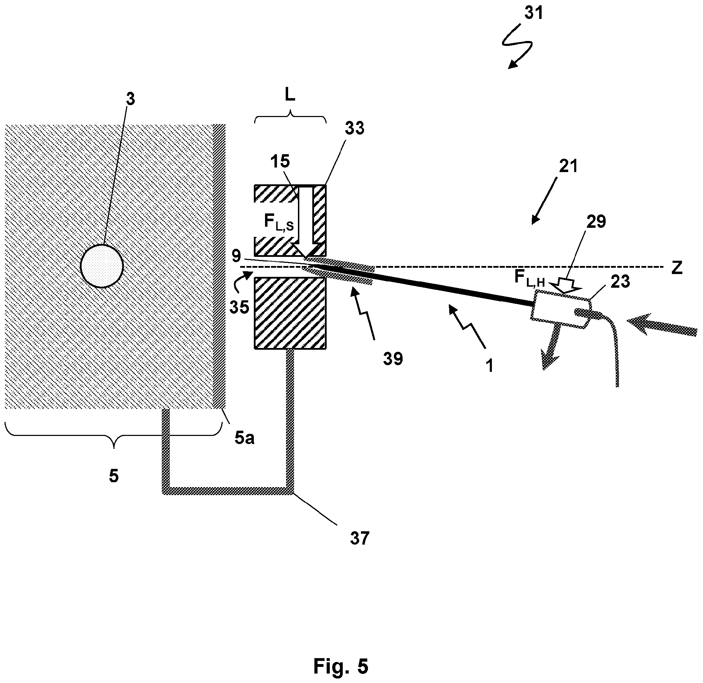

Fig. 5 zeigt wie die Schutzhülse 39 die Nadelspitze 9 vor der Einwirkung der großen seitlichen Kraft $F_{L,S}$ 15 schützt, die bereits durch eine relativ geringe manuelle Verkantungskraft $F_{L,H}$ 29 auf das Handgriffelement 23 bewirkt wird. Die axiale Länge der Schutzhülse 39 übersteigt die axiale Länge der Nadelspitze 9 mindestens um das Doppelte, hier um ein Vielfaches. Die axiale Länge der Schutzhülse 39 entspricht mindestens der Hälfte der Länge L der Aufnahme 35, hier sind sie im Wesentlichen gleich.

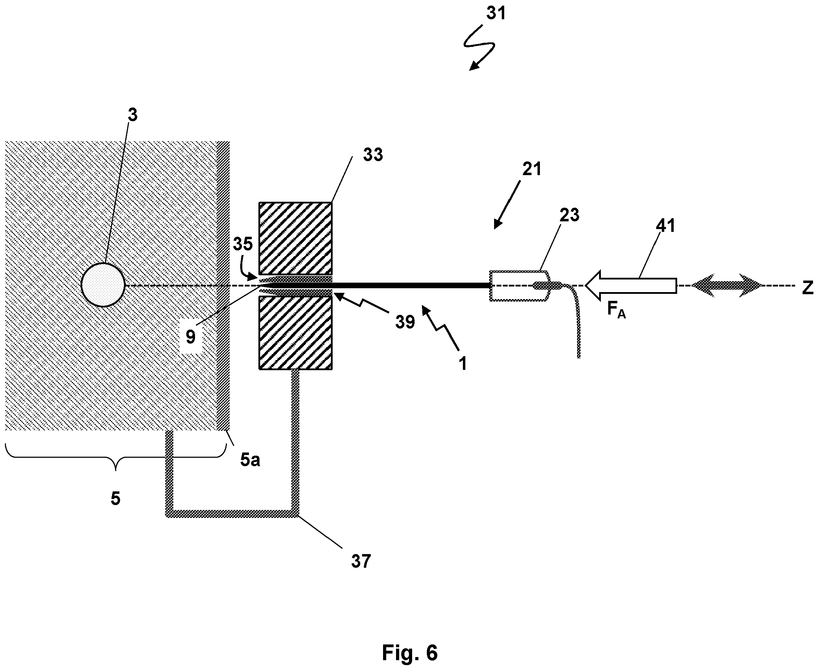

In Fig. 6 ist der Einführabschnitt 1 zusammen mit der Schutzhülse 39 in die Aufnahme 35 des Positionierungselements 33 eingeführt, sodass der Lichtapplikator 21 genau senkrecht zur Haut 5a in Z-Richtung ausgerichtet ist. Die Schutzhülse 39 befindet sich relativ zum Einführabschnitt 1 noch in der ersten Axialposition, in welcher sie die Nadelspitze 9 schützend umschließt.

Zum weiteren Vorstechen des Einführabschnitts 1 muss die Bedienperson nur manuell eine axiale Kraft $F_A$ auf das Handgriffelement 23 ausüben. Etwaige manuelle Verkantungskräfte $F_{L,H}$ 29 auf das Handgriffelement 23 würden nur eine Verbiegung des Einführabschnitts 1 bewirken, aber nicht mehr als seitliche Kraft $F_{L,S}$ 15 auf die von der Schutzhülse 39 geschützte Nadelspitze 9.

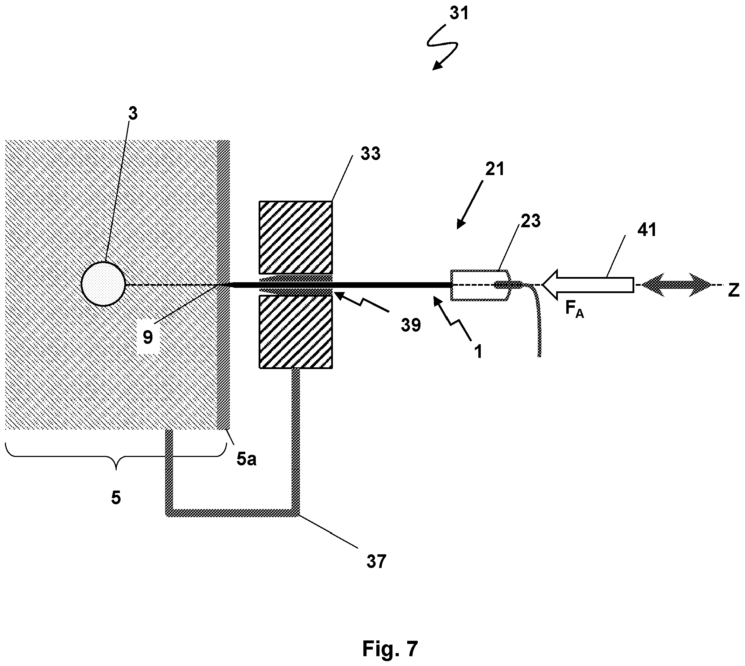

In Fig. 7 ist die Nadelspitze 9 des Einführabschnitts 1 bereits in die Haut 5a eingestochen. Die Schutzhülse 39 ist dabei allerdings in der in Fig. 6 gezeigten Position in der Aufnahme 35 des Positionierungselements 33 verblieben. Die Schutzhülse 39 kann beispielsweise einen Anschlag (in Fig. 7 nicht gezeigt) aufweisen, der eine maximal distalwärtige Position der Schutzhülse 39 in der Aufnahme 35 des Positionierungselements 33 definiert, indem der Anschlag gegen das Positionierungselement 33 anschlägt. Durch das distalwärtige Herausdrücken des Einführabschnitts 1 aus der Schutzhülse 39 hat die Schutzhülse 39 bezüglich des Einführabschnitts 1 die erste Axialposition verlassen und befindet sich nun in einer zweiten Axialposition, in der die Schutzhülse 39 gegenüber der Nadelspitze 9 axial zurückgezogen ist. Es kann dabei viele zweite Axialpositionen geben, wobei die zweite Axialposition von der Einstichtiefe der Nadelspitze 9 in den Körper 5 abhängt, deren Maximum lediglich durch die Länge des Einführabschnitts 1 begrenzt ist, d.h. erreicht würde, wenn das Handgriffelement 23 an das Positionierungselement 33 stieße.

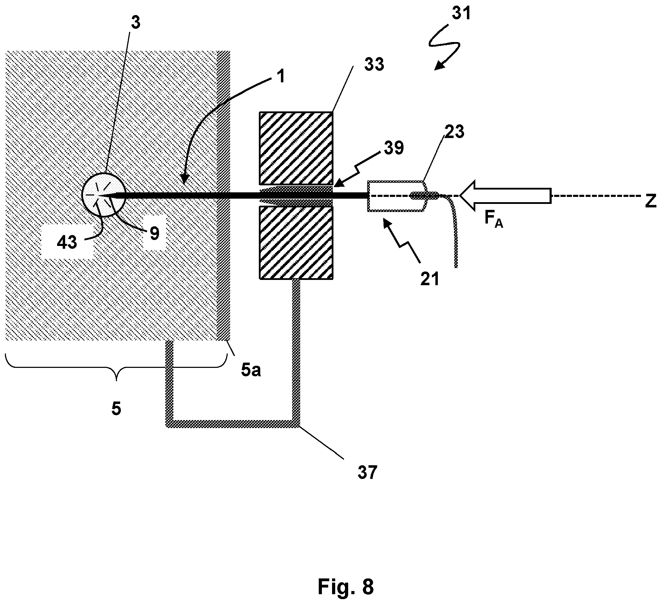

In Fig. 8 ist die Nadelspitze 9 in das Gewebe 3 eingestochen und hat die axiale Zielposition erreicht, in der die PDD oder PDT durchgeführt werden kann, d.h. die LED 7 wird eingeschaltet und die Nadelspitze 9 mit dem lichttransparenten Streukörper streut das Licht 43 der LED 7 in einen möglichst großen Raumwinkel, vorzugsweise von deutlich über $3\pi$, in das Gewebe 3. Das Gewebe 3 kann beispielsweise ein Tumor oder anderes pathologisches Gewebe sein, das mit oder ohne Hilfe eines dort angereicherten Photosensibilisators auf das Licht 43 der LED 7 reagiert, wobei lokal begrenzt toxische Substanzen, z.B. Sauerstoffradikale, entstehen, die das pathologische Gewebe 3 schädigen. Da dieser Prozess einer gewissen Bestrahlungsdauer des Lichts bedarf, wird der Einführabschnitt 1 vorzugsweise gegenüber dem Positionierungselement 33 in dieser gewählten zweiten Axialposition fixiert. Dazu kann beispielsweise ein hier nicht gezeigtes Klemmelement die Schutzhülse 39 mit dem Einführabschnitt 1 verklemmen. Zusätzlich kann das Klemmelement auch die Schutzhülse 39 mit dem Positionierungselement 33 verklemmen.

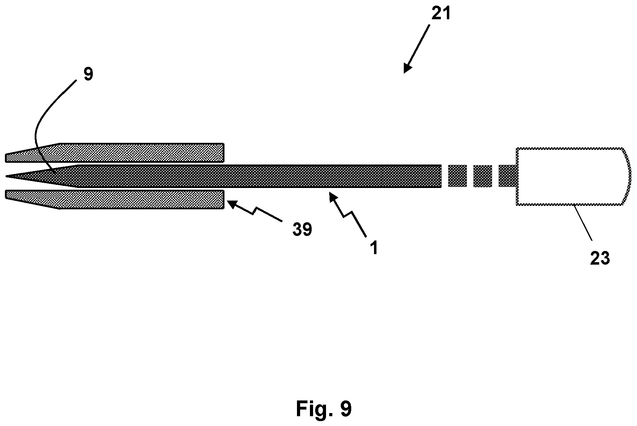

Fig. 9 zeigt den Lichtapplikator 21 schematisch vergrößert, wobei er bei einer maßstabsgetreuen Vergrößerung der Länge nach nicht auf das Papier gepasst hätte und daher in der Länge verkürzt dargestellt ist. Die Schutzhülse 39 weist einen Innendurchmesser auf, der passgenau auf den Außendurchmesser des Einführabschnitts 1 passt. Die Schutzhülse 39 umschließt die Nadelspitze 9 schützend in der gezeigten ersten Axialposition relativ zum Einführschnitt 1. Das Handgriffelement 23 befindet sich am proximalen Ende des Lichtapplikators 21. Am distalen Ende der Schutzhülse 39 ist diese außenseitig gefast, d.h. der Außendurchmesser verjüngt sich distalwärts, sodass die Schutzhülse leichter in die Aufnahme 35 des Positionierungselements 33 eingeführt werden kann.

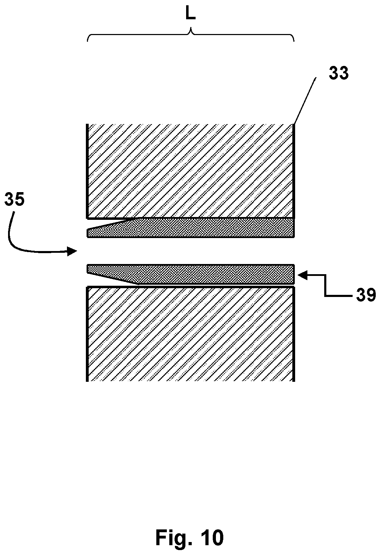

In Fig. 10 ist die Schutzhülse 39 in der Aufnahme 35 des Positionierungselements 33 gezeigt, in der sie passgenau sitzt. Hier ist die Länge der Schutzhülse 39 im Wesentlichen gleich der Dicke L des Positionierungselements, welche der axialen Länge L der Aufnahme 35 entspricht, wodurch eine gute Führung erreicht wird.

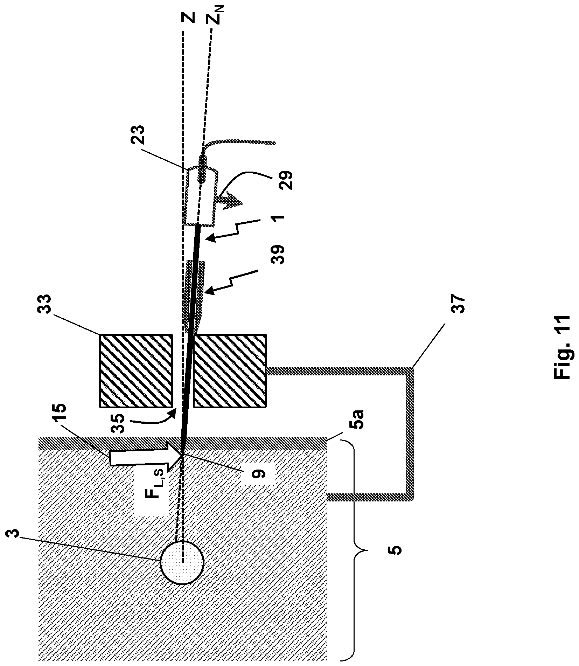

Fig. 11 zeigt eine Problematik, wenn die Schutzhülse 39 nicht korrekt in der Aufnahme 35 des Positionierungselements 33 sitzt und zugleich die erste Axialposition verlassen hat. Dies kann beispielsweise beim Einführen passieren, wenn die Schutzhülse 39 mit dem distalen Ende gegen das Positionierungselement 33 stößt und die Nadelspitze 9 distalwärts aus der Schutzhülse 39 gedrückt wird. Ebenso kann diese Situation beim proximalwärtigen Zurückziehen des Lichtapplikators 21 entstehen, wenn die Schutzhülse 39 vorzeitig proximalwärts aus der Aufnahme 35 des Positionierungselements 33 gezogen wird, bevor die Nadelspitze 9 so weit in die Schutzhülse 39 gezogen wurde, dass die Schutzhülse 39 die schützende erste Axialposition innehat. Da der Innendurchmesser der Aufnahme 35 an den Außendurchmesser der Schutzhülse 39 angepasst ist, der größer als der Außendurchmesser des Einführelements 1 ist, hat das Einführelement 1 ohne die korrekt positionierte Schutzhülse 39 ein erhebliches seitliches Spiel in der Aufnahme 35. Wie in Fig. 11 gezeigt, kann der Lichtapplikator 21 dann verkantet werden, sodass die Längsachse $Z_N$ nicht mehr mit der vorgesehenen Einstichrichtung Z fluchtet. Dadurch können wie oben beschrieben erhebliche seitliche Kräfte $F_{L,S}$ 15 auf die Nadelspitze 9 wirken, die zum Abriss oder Abbruch der Nadelspitze 9 führen können.

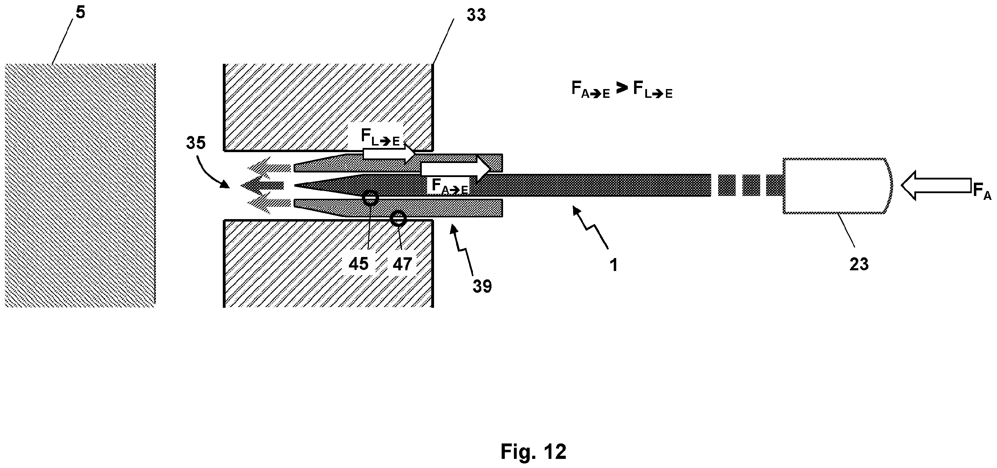

In den Figuren 12 bis 17 sind die axialen Kräfteverhältnisse verdeutlicht, die vorzugsweise bei dem hierin offenbarten Lichtapplikatorsystem 31 vorherrschen. Bei dem in Fig. 12 gezeigten Einführen der Schutzhülse 39 in die Aufnahme 35 verbleibt die Schutzhülse 39 in der ersten Axialposition. Um dies zu erreichen, weist die Schutzhülse 39 nach innen eine erste mechanische Schnittstelle 45 zum Einführabschnitt 1 und nach außen eine zweite mechanische Schnittstelle 47 zum Positionierungselement 33 auf. Die Schnittstellen 45, 47 können beispielsweise Reibungsflächen oder Reibungspunkte sein. Die Schnittstellen 45, 47 erzeugen jeweils eine der axialen Einstichkraft $F_A$ entgegengesetzte Widerstandskraft, beispielsweise eine Haft- und/oder Gleitreibungskraft. Solange die Schutzhülse 39 noch nicht die finale maximal distale Position in der Aufnahme 35 erreicht hat, ist die von der zweiten Schnittstelle 47 erzeugte Widerstandskraft $F_{L \to E}$ kleiner als die Einstichkraft $F_A$. Die von der ersten Schnittstelle 45 erzeugte Widerstandskraft $F_{A \to E}$ ist hingegen größer als die von der zweiten Schnittstelle 47 erzeugte Widerstandskraft $F_{L \to E}$. Daher verbleibt die Schutzhülse 39 in der ersten Axialposition bezüglich des Einführabschnitts 1 und überträgt die Einstichkraft $F_A$ mittels der Widerstandskraft $F_{A \to E}$ auf die Schutzhülse 39. Daher bewegt sich die Schutzhülse 39 distalwärts in die Aufnahme 35 entgegen der kleineren Widerstandskraft $F_{L \to E}$.

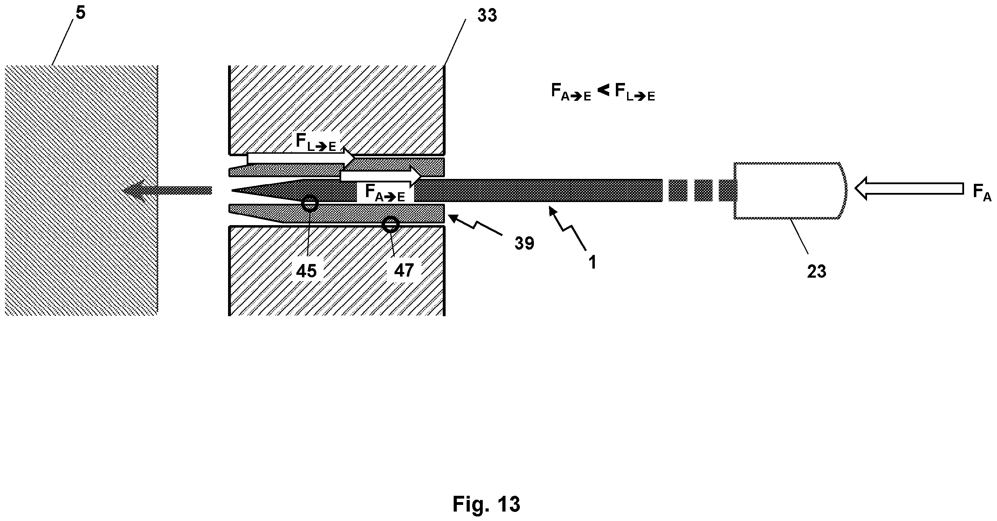

In Fig. 13 ist der Moment gezeigt, in dem die Schutzhülse 39 die finale Distalposition, d.h. die Sollposition, in der Aufnahme 35 erreicht hat, und sich zugleich noch in der ersten Axialposition bezüglich des Einführabschnitts 1 befindet. Die Schutzhülse 39 kann beispielsweise gerade mit einem Anschlag (nicht gezeigt) gegen das Positionierungselement 33 gestoßen sein. Der Anschlag könnte als Teil der zweiten Schnittstelle 47 aufgefasst werden, wobei sich die proximalwärtige Widerstandskraft $F_{L \to E}$ beim Anschlag sofort so weit erhöht, dass sie größer als die proximalseitige Widerstandskraft $F_{A \to E}$ wird. Die Schutzhülse 39 kann also nicht weiter distalwärts gedrückt werden, da das Positionierungselement 33 relativ zum Körper 5 fixiert ist. Die Bedienperson muss die Einstichkraft $F_A$ gegenüber Figur 12 erhöhen, um die größere Widerstandskraft $F_{A \to E}$ der ersten Schnittstelle 45 zu überwinden, um die Nadelspitze 9 distalwärts aus der Schutzhülse 39 zu drücken.

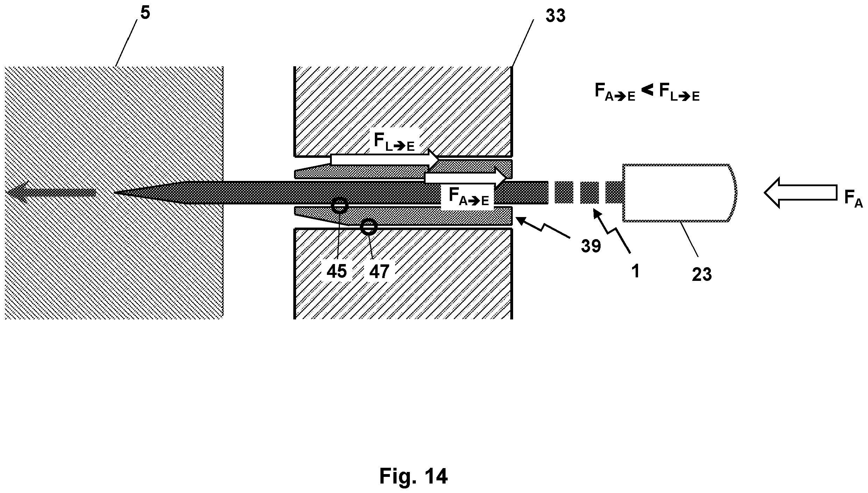

In Fig. 14 hat die Schutzhülse 39 die erste Axialposition verlassen, indem der Einführabschnitt 1 distalwärts aus der in maximaler Distalposition, d.h. der Sollposition, befindlichen Schutzhülse 39 herausgedrückt wird. Sobald eine in der ersten Axialposition anfänglich hohe Widerstandskraft $F_{A \to E}$ der ersten Schnittstelle 45 überwunden ist, kann die Widerstandskraft $F_{A \to E}$ außerhalb der ersten Axialposition geringer sein, sodass beim weiteren Vorstechen, wie in Fig. 14 gezeigt, die Einstichkraft $F_A$ wieder geringer sein kann. Beispielsweise kann die anfänglich hohe Widerstandskraft $F_{A \to E}$ eine Haftreibungskraft und/oder eine elastische Verformungskraft beinhalten. Die Widerstandskraft $F_{A \to E}$ kann dann geringer werden, wenn die Haftreibungskraft in eine geringere Gleitreibungskraft übergeht und/oder keine elastische Verformungskraft mehr benötigt wird. Während und nach dem Einstich in die Haut 5a und den Körper 5 wirken zusätzliche Widerstandskräfte der Einstichkraft $F_A$ entgegen, die entsprechend groß sein muss, damit die Nadelspitze 9 bis zum Gewebe 3 eingestochen werden kann. Um den Einstich so minimalinvasiv wie möglich zu gestalten, ist der Einführabschnitt 1 allerdings in den Grenzen der Stabilität so dünn wie möglich und die Nadelspitze 9 so spitz wie möglich ausgeführt, sodass die von der Haut 5a und/oder dem Körper 5 induzierten Widerstandskräfte möglichst minimal sind.

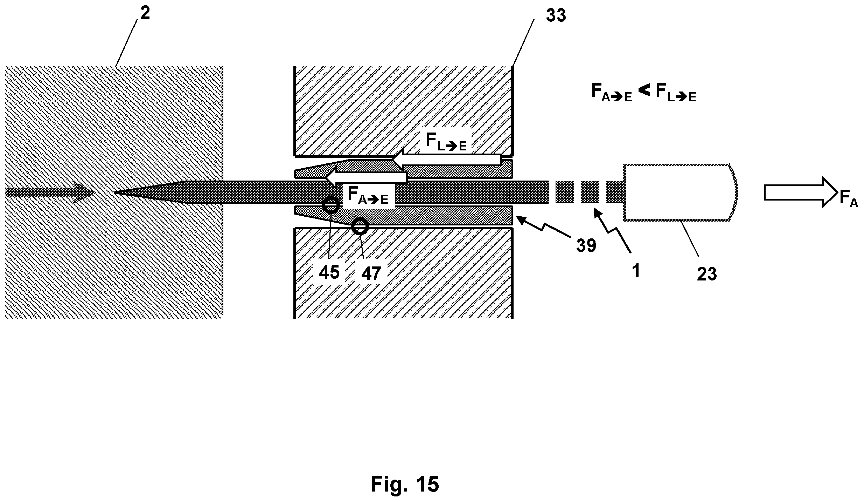

In Fig. 15 wird der Einführabschnitt 1 nach erfolgter PDD oder PDT proximalwärts aus dem Körper 5 zurückgezogen. Die Schutzhülse 39 ist hier in der maximalen Distalposition eingerastet, sodass die von der zweiten Schnittstelle 47 erzeugte distalwärtige Widerstandskraft $F_{L \to E}$ höher ist als die von der ersten Schnittstelle 45 erzeugte distalwärtige Widerstandskraft $F_{A \to E}$. Dadurch verbleibt die Schutzhülse 39 in der maximalen Distalposition, d.h. Sollposition, in der Aufnahme 35 des Positionierungselements 33 während die Nadelspitze 9 wieder proximalwärts in die Schutzhülse 39 eingefahren wird.

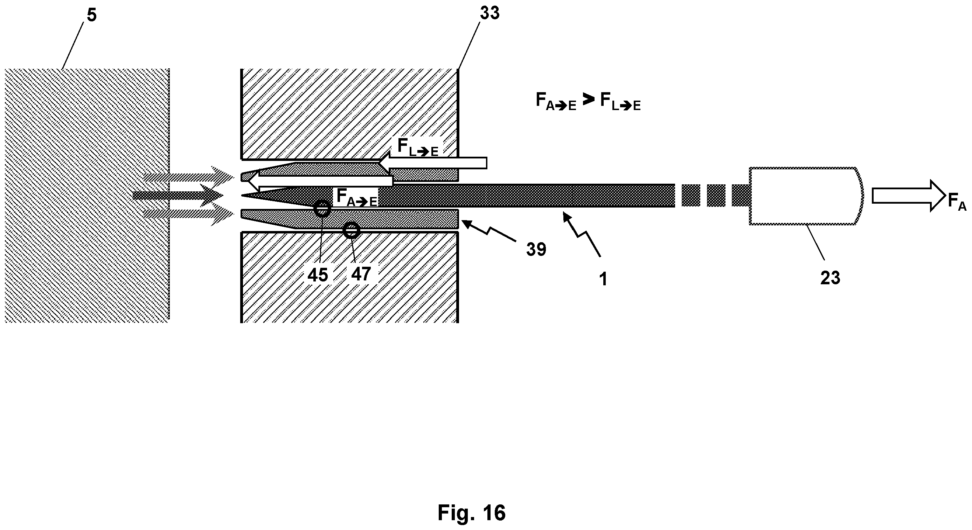

In Fig. 16 ist die Nadelspitze 9 maximal proximalwärts in die Schutzhülse 39 eingefahren, sodass die Schutzhülse 39 wieder die schützende erste Axialposition bezüglich des Einführabschnitts 1 innehat. Die erste Schnittstelle 45 kann einen Anschlag aufweisen, der bei maximaler Proximalposition des Einführabschnitts 1 bezüglich der Schutzhülse 39 proximalwärts anschlägt, sodass die distalwärtige Widerstandskraft $F_{A \to E}$ größer als die von der eingerasteten zweiten Schnittstelle 47 erzeugte distalwärtige Widerstandskraft $F_{L \to E}$ ist. Die Bedienperson muss die proximalwärtige Herausziehkraft $F_A$ erhöhen, um die distalwärtige Widerstandskraft $F_{L \to E}$ zu überwinden und die zweite Schnittstelle 47 aus der Einrastung zu lösen. Die Schutzhülse 39 verlässt dann proximalwärts ihre Sollposition in der Aufnahme 35 und verbleibt bezüglich des Einführabschnitts 1 in der ersten Axialposition.

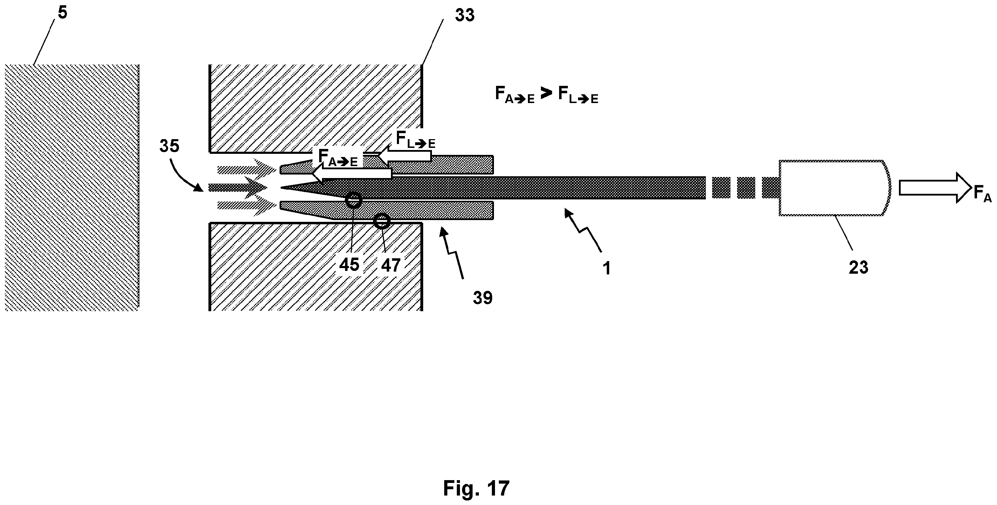

Sobald die Einrastung der zweiten Schnittstelle 47, wie in Fig. 17 gezeigt, gelöst ist, wirkt der Herausziehkraft $F_A$ eine geringere Gleitreibungskraft $F_{L \to E}$ der zweiten Schnittstelle 47 entgegen, sodass die Schutzhülse 39, die sich in der schützenden ersten Axialposition bezüglich des Einführabschnitts 1 befindet, einfach proximalwärts aus der Aufnahme 35 gezogen werden kann. Die Nadelspitze 9 bleibt durch die sich in der ersten Axialposition befindliche Schutzhülse 39 geschützt. Ebenso schützt die Schutzhülse 39 die Bedienperson und/oder Patienten vor unbeabsichtigtem Stechen mit der Nadelspitze 9.

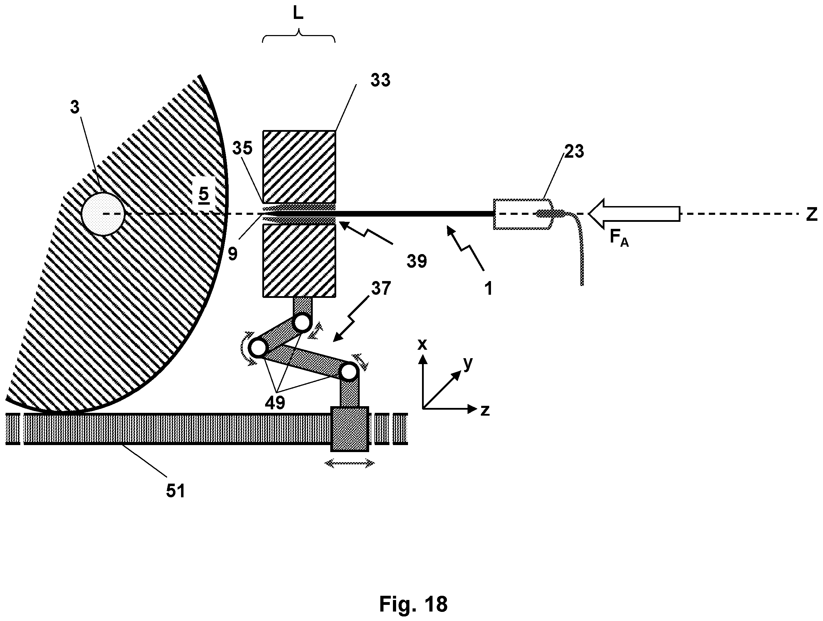

Fig. 18 zeigt die Fixierung des Positionierungselements 33 relativ zum Körper 5 des Patienten genauer. Das Fixierelement 37 weist hier einen Gelenkarm mit mehreren Gelenken 49 auf, wobei das Positionierungselement 33 in vorzugsweise sechs Freiheitsgraden, d.h. in drei Raumrichtungen x,y,z beliebig positioniert und drei Winkeleinstellungen beliebig ausgerichtet, fixiert werden kann. Ein Behandlungstisch 51 dient hier als örtlicher Referenzkörper, bezüglich welches das Positionierungselement 33 positioniert und ausgerichtet fixiert werden kann. Der Körper 5 des Patienten ist vorzugsweise ebenfalls auf dem Behandlungstisch 51 fixiert und/oder nimmt eine fest definierte Position darauf oder daran ein. Das Positionierungselement 33 kann mit einer distalen Seite auf der Haut 5a des Körpers 5 aufliegen. Vorzugsweise ist das Positionierungselement 33 sogar auf die Haut 5a geklebt.

Das Positionierungselement 33 kann eine gelochte Kunststoffplatte in Form einer Lochplatte oder einem Lochraster sein. Um die Ausrichtung der Schutzhülse 39 definiert führen zu können, ist es von Vorteil, wenn sich die Aufnahme 35 über eine gewisse Länge L in Z-Richtung erstreckt. Dazu kann das Positionierungselement 33 eine entsprechende Dicke L aufweisen. Alternativ dazu kann jede Aufnahme 35 einen sich in proximale Z-Richtung erstreckenden Hülsenfortsatz aufweisen, der fest oder lösbar mit dem Positionierungselement 33 verbunden ist, um das Positionierungselement 33 nicht unnötig massiv und schwer auszugestalten. Allerdings kann für bestimmte Anwendungen eine massive und schwere Ausgestaltung des Positionierungselements 33 auch vorteilhaft sein.

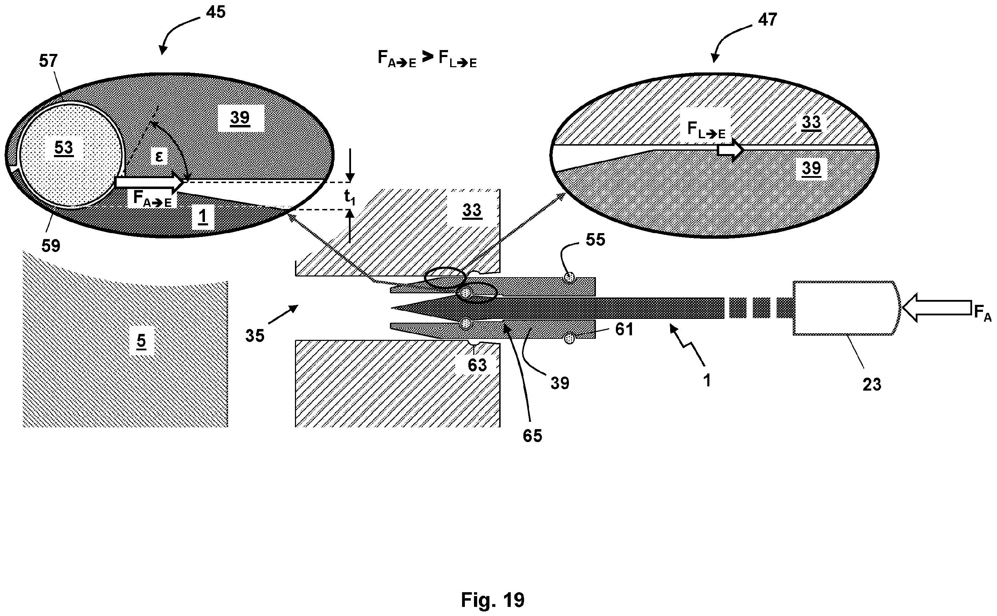

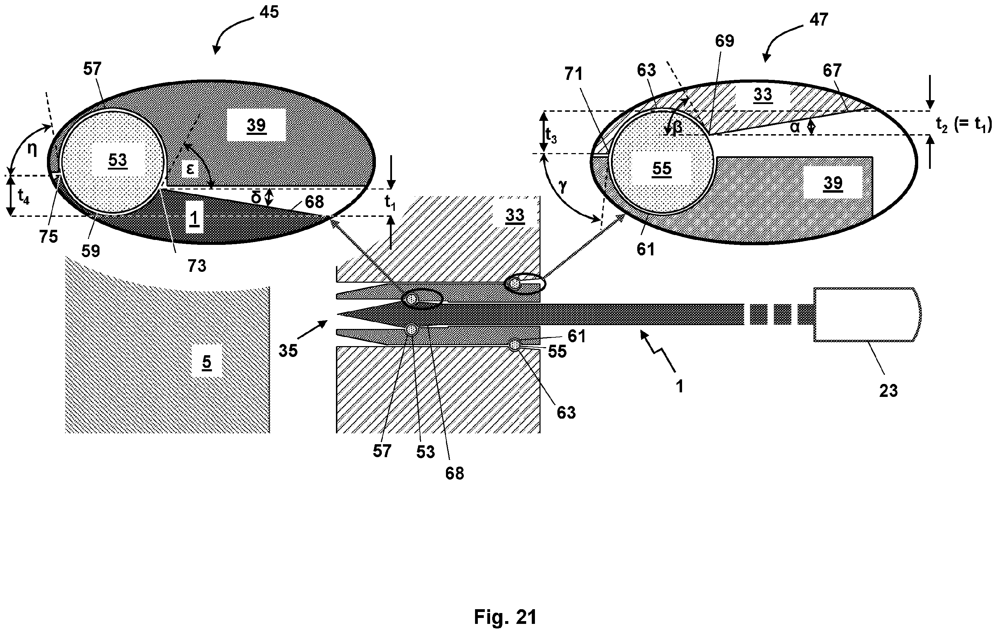

In Figuren 19 bis 26 ist die Funktionsweise der Schnittstellen 45, 47 genauer an einem Ausführungsbeispiel erläutert. In dem gezeigten Ausführungsbeispiel weisen die Schnittstellen 45, 47 jeweils einen oder mehr elastisch verformbare Eingriffselemente 53, 55, hier in Form von O-Ringen, auf. Ein erstes Eingriffselement 53 der ersten Schnittstelle 45 sitzt zwischen dem Einführabschnitt 1 und der Schutzhülse 39 mit einem radial äußeren Teil in einer innenseitig an der Schutzhülse 39 angeordneten und radial umlaufenden ersten Nut 57. Ein radial innen liegender Teil des ersten Eingriffselements 53, der hier kleiner ist als der radial äußere Teil, liegt in der in Fig. 19 gezeigten ersten Axialposition der Schutzhülse 39 in einer außenseitig am Einführabschnitt 1 angeordneten und radial umlaufenden ersten Gegennut 59. Ein zweites Eingriffselement 55 der zweiten Schnittstelle 47 sitzt zwischen der Schutzhülse 39 und dem Positionierungselement 33 mit einem radial inneren Teil in einer außenseitig an der Schutzhülse 39 angeordneten und radial umlaufenden zweiten Nut 61. Ein radial außen liegender Teil des zweiten Eingriffselements 55, der hier kleiner ist als der radial innere Teil, liegt in der in Fig. 21 bis 25 gezeigten maximalen Distalposition, d.h. Sollposition, der Schutzhülse 39 bezüglich der Aufnahme 35 in einer innenseitig an der Aufnahme 35 des Positionierungselements 33 angeordneten und radial umlaufenden zweiten Gegennut 63. Sobald sich die Eingriffselemente 53, 55 in den jeweiligen Gegennuten 59, 63 befinden, sind die entsprechenden Schnittstellen 45, 47 "eingerastet", d.h. die entsprechende Widerstandskraft $F_{L \to E}$ bzw. $F_{A \to E}$ ist temporär erhöht.

Beim Einschieben der Schutzhülse 39 in die Aufnahme 35 ist, wie in Fig. 19 gezeigt, das erste Eingriffselement 53 in die Gegennut 59 eingerastet und das zweite Eingriffselement 55 außerhalb der zweiten Gegennut 63. Daher ist die proximalwärtige Widerstandskraft $F_{A \to E}$ der ersten Schnittstelle 45 höher als die proximalwärtige Widerstandskraft $F_{L \to E}$ der zweiten Schnittstelle 47, die sich lediglich aus der Gleitreibungskraft zwischen Schutzhülse 39 und Aufnahme 35 ergibt. Auf das eingerastete erste Eingriffselement 53 wirkt daher distalwärts nur der Teil der Einstichkraft $F_A$, der der Gleitreibungskraft zwischen Schutzhülse 39 und Aufnahme 35 entspricht. Dieser Kraftanteil reicht allerdings nicht aus, um das erste Eingriffselement 53 so weit radial nach außen zusammen zu drücken, dass sich die Gegennut 59 distalwärts löst. Die proximalseitige radiale Eindringtiefe $t_1$ des ersten Eingriffselements 53 in die erste Gegennut 59 ist entsprechend so gewählt, dass vom Einführabschnitt 1 eine bestimmte distalwärtige Mindestkraft auf das erste Eingriffselement 53 ausgeübt werden muss, um dieses so weit radial nach außen zusammen zu drücken, dass sich die Gegennut 59 distalwärts löst. Unterhalb dieser Mindestkraft ist der Einführabschnitt 1 in der sich in der ersten Axialposition befindlichen Schutzhülse 39 distalwärts gesichert. Distalwärts ist die Schutzhülse 39 unverlierbar dadurch am Einführabschnitt 1 gesichert, dass dieser einen innenseitigen Anschlag 65 der Schutzhülse 39 mittels eines Vorsprungs, einer Auskragung oder einer Verdickung hintergreift. Alternativ oder zusätzlich ist die Gegennut 59 derart asymmetrisch ausgestaltet, dass sich die Gegennut 59 nur distalwärts, aber nicht proximalwärts vom ersten Eingriffselement 53 lösen kann.

Die Widerstandskraft $F_{A \to E}$ der ersten Schnittstelle 45 hängt nicht nur von der proximalseitigen radialen Eindringtiefe $t_1$ des ersten Eingriffselements 53 in die erste Gegennut 59 ab, sondern auch von der Geometrie des ersten Eingriffselements 53 sowie der Geometrie der ersten Gegennut 59. Hier sind die Eingriffselemente 53, 55 als O-Ringe ausgebildet und die Querschnittsgeometrie der Gegennuten 59, 63 ist kreisbogenförmig mit entsprechendem Durchmesser zur passgenauen Aufnahme des jeweiligen O-Rings 53, 55. Die Widerstandskraft $F_{A \to E}$ der ersten Schnittstelle 45 hängt auch von einem in Fig. 19 gezeigten Rampenwinkel $\varepsilon$ ab, der zwischen 0° und 90° liegen kann, wobei die Widerstandskraft $F_{A \to E}$ für 90° maximal und für 0° minimal ist. Der Rampenwinkel $\varepsilon$ ist entsprechend so gewählt, dass vom Einführabschnitt 1 eine bestimmte distalwärtige Mindestkraft auf das erste Eingriffselement 53 ausgeübt werden muss, um dieses so weit radial nach außen zusammen zu drücken, dass sich die Gegennut 59 distalwärts löst.

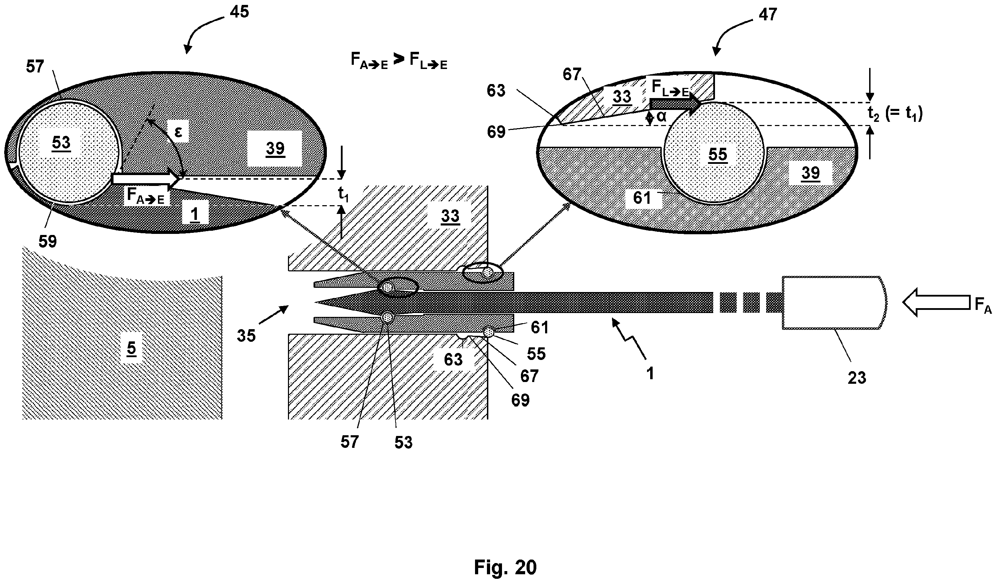

In Fig. 20 ist die Schutzhülse 39 so weit distalwärts in die Aufnahme 35 eingeschoben, dass das zweite Eingriffselement 55 gerade an das Positionierungselement 33 anschlägt. Die erste Schnittstelle 45 mit dem ersten Eingriffselement 53 ist gegenüber Fig. 19 unverändert. Das proximale Ende der Aufnahme 35 weitet sich proximalwärts auf und bildet somit eine konische Rampenfläche 67. Damit ist im Zusammenhang mit dem am distalen Ende der Schutzhülse verjüngten Außendurchmesser der Schutzhülse 39 nicht nur das Einführen der Schutzhülse 39 in die Aufnahme 35 leichter, sondern die Rampenfläche 67 dient zudem dem Zusammendrücken des zweiten Eingriffselements 55 radial nach innen. Dadurch wird die proximalwärtige Widerstandskraft $F_{L \to E}$ der zweiten Schnittstelle 47 in definierter Weise unterhalb der proximalwärtigen Widerstandskraft $F_{A \to E}$ der ersten Schnittstelle 45 gehalten.

Ein proximales Ende 69 der zweiten Gegennut 63, welches hier zugleich ein distales Ende der Rampenfläche 67 ist, bestimmt eine proximalseitige radiale Eindringtiefe $t_2$, um die das zweite Eingriffselement 55 radial nach innen zusammengedrückt werden muss, bevor es sich in die Gegennut 63 nach außen wieder ausdehnen kann, d.h. dort einrastet. Wenn die O-Ringe 53, 55 den gleichen Querschnitt haben und gleiches Material aufweisen, kann der Betrag $t_2$ dem Betrag $t_1$ der ersten Schnittstelle 45 entsprechen. Damit sich allerdings beim Zusammendrücken des zweiten Eingriffselements 55 nicht die erste Schnittstelle 45 löst, d.h. das erste Eingriffselement 53 zusammengedrückt wird, ist ein Rampenwinkel $\alpha$ der Rampenfläche 67 flacher gewählt als der Rampenwinkel $\varepsilon$ der ersten Schnittstelle 45, d.h. $\alpha < \varepsilon$. Dadurch ist sichergestellt, dass die Widerstandkraft $F_{A \to E}$ der ersten Schnittstelle 45 solange größer als die Widerstandkraft $F_{L \to E}$ der zweiten Schnittstelle 47 ist bis die Schutzhülse 39 die maximale Distalposition, d.h. die Sollposition, in der Aufnahme 35 erreicht hat und das zweite Eingriffselement 55 in die zweite Gegennut 63 eingerastet ist.

Fig. 21 zeigt beide Schnittstellen 45, 47 in eingerastetem Zustand. Die Schutzhülse 39 befindet sich sowohl in der ersten Axialposition bezüglich des Einführabschnitts 1 als auch in der maximalen Distalposition in der Aufnahme 35, d.h. in der Sollposition, da die erste Schnittstelle 45 mittels des ersten Eingriffselement 53 in der ersten Gegennut 59 eingerastet ist und die zweite Schnittstelle 47 mittels des zweiten Eingriffselements 55 in der zweiten Gegennut 63 eingerastet ist. Die Gegennuten 59, 63 sind jeweils derart asymmetrisch ausgebildet, dass sich die jeweiligen Widerstandkräfte $F_{A \to E}$ bzw. $F_{L \to E}$ beim Verlassen der eingerasteten Position in distale Richtung und in proximale Richtung voneinander unterscheiden.

Die zweite Schnittstelle 47 kann beispielsweise einen Anschlag dadurch ausbilden, dass sich ein distales Ende 71 der zweiten Gegennut 63 so weit radial nach innen erstreckt, dass sich eine distalseitige radiale Eindringtiefe $t_3$ des zweiten Eingriffselements 55 in die zweite Gegennut 63 ergibt, wobei $t_3 > t_2$. Das zweite Eingriffselement 55 kann dabei so beschaffen sein, dass es faktisch nicht um die Eindringtiefe $t_3$ zusammengedrückt werden kann oder dies nur bei unsachgemäß hoher Krafteinwirkung möglich wäre. Außerdem definiert die zweite Gegennut 63 einen relativ steilen distalseitigen Rampenwinkel $\gamma$, wobei $\gamma > \varepsilon$. Damit bildet das zweite Eingriffselement 55 in der zweiten Gegennut 63 einen distalwärtigen Anschlag, der die maximale Distalposition, d.h. die Sollposition, der Schutzhülse 39 in der Aufnahme 35 bestimmt.

Die erste Schnittstelle 45 ist in analoger Weise umgekehrt asymmetrisch ausgestaltet. Die proximalseitige radiale Eindringtiefe $t_1$ des ersten Eingriffselements 53 in die erste Gegennut 59 wird von einem proximalen Ende 73 der ersten Gegennut 59 bestimmt. Eine distalseitige radiale Eindringtiefe $t_4$ des ersten Eingriffselements 53 in die erste Gegennut 59 wird von einem distalen Ende 75 der ersten Gegennut 59 bestimmt, wobei $t_4 > t_1$. Die Beträge $t_1$, $t_2$, $t_3$ und $t_4$ können so gewählt werden, dass $t_4 = t_3 > t_2 = t_1$ gilt. Das erste Eingriffselement 53 kann dabei so beschaffen sein, dass es faktisch nicht um die Eindringtiefe $t_4$ zusammengedrückt werden kann oder dies nur bei unsachgemäß hoher Krafteinwirkung möglich wäre. Außerdem definiert die erste Gegennut 59 einen relativ steilen distalseitigen Rampenwinkel $\eta$, wobei $\eta > \varepsilon$. Damit bildet das erste Eingriffselement 53 in der ersten Gegennut 59 einen proximalwärtigen Anschlag, der die Schutzhülse 39 unverlierbar am Einführabschnitt 1 sichert. Der Einführabschnitt 1 bildet zudem außenseitig eine Rampenfläche 68 analog zur Rampenfläche 67 der zweiten Schnittstelle 47. Die Rampenfläche 68 erstreckt sich von der ersten Gegennut 59 proximalwärts, sodass sich der Einführabschnitt 1 mit einem Rampenwinkel $\delta$, der gleich dem Rampenwinkel $\alpha$ sein kann, über eine gewisse Strecke etwa um den Betrag $t_1$ proximalwärts verjüngt. Der Rampenwinkel $\delta$ ist so flach gewählt, dass die Widerstandskraft $F_{A \to E}$ der ersten Schnittstelle 45 beim Zurückziehen der Nadelspitze 9 in die Schutzhülse 39 kleiner ist als die Widerstandkraft $F_{L \to E}$ der eingerasteten zweiten Schnittstelle 47 und somit die erste Schnittstelle 45 einrastet, bevor die zweite Schnittstelle 47 ausrastet und die Schutzhülse 39 die Sollposition proximalwärts verlässt.

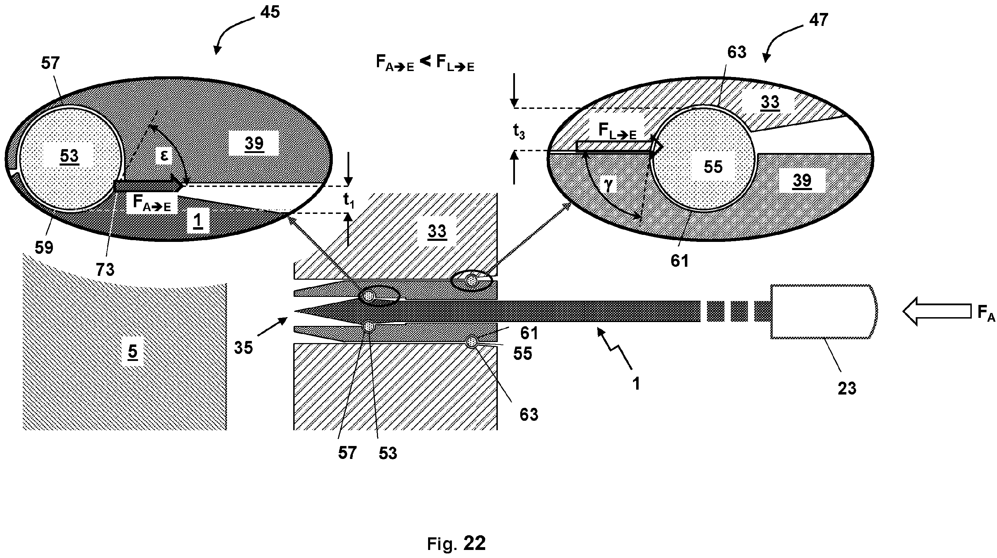

Fig. 22 zeigt die sich ergebenden Widerstandkräfte $F_{A \to E}$ und $F_{L \to E}$, wenn in der eingerasteten Position von Fig. 21 eine distalwärtige Einstichkraft $F_A$ auf das Handgriffelement 23 ausgeübt wird. Die proximalwärtige Widerstandkraft $F_{L \to E}$ der zweiten Schnittstelle 47 ist nun höher als die proximalwärtige Widerstandkraft $F_{A \to E}$ der ersten Schnittstelle 45 (weil $t_3 > t_1$ und $\gamma > \varepsilon$). Dadurch drückt die erste Gegennut 59 mit einer proximalseitigen Rampenfläche mit dem Rampenwinkel $\varepsilon$ gegen das eingerastete erste Eingriffselement 53, sodass sich das proximale Ende 73 der ersten Gegennut 59 unter elastischer Verformung des ersten Eingriffselements 53 distalwärts daran vorbeidrückt.

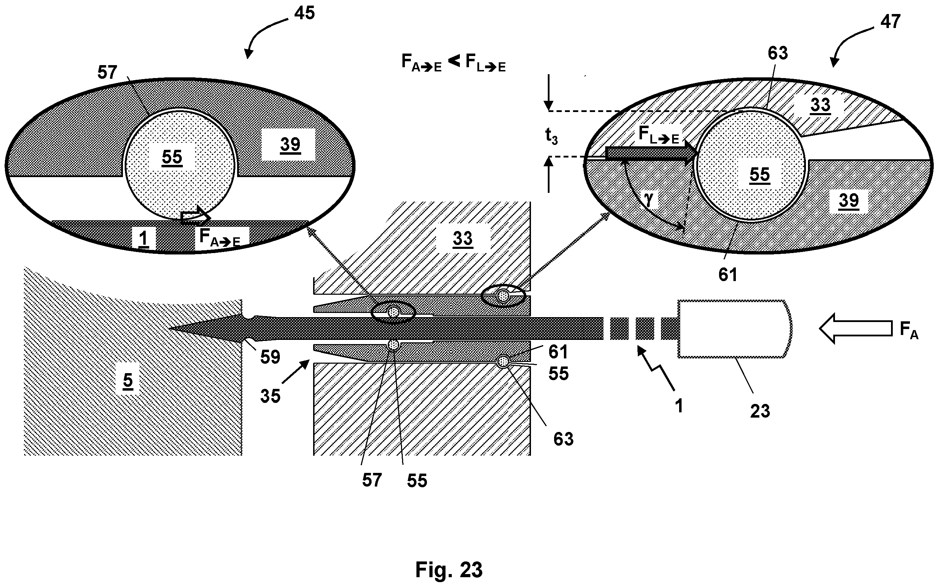

In Fig. 23 ist das proximale Ende 73 der ersten Gegennut 59 unter elastischer Verformung des ersten Eingriffselements 53 distalwärts daran vorbeidrückt und somit die anfänglich höhere Widerstandkraft $F_{A \to E}$ der ersten Schnittstelle 45 überwunden. Die Schutzhülse 39 verbleibt in der maximalen Distalposition bezüglich der Aufnahme 35, aber hat die erste Axialposition bezüglich des Einführabschnitts 1 dadurch verlassen, dass der Einführabschnitt 1 distalwärts weitergedrückt wurde. Nunmehr bildet nur noch eine relativ geringe Gleitreibungskraft die Widerstandkraft $F_{A \to E}$ der ersten Schnittstelle 45, sodass der Einführabschnitt 1 relativ leicht in den Körper 5 eingestochen werden kann.

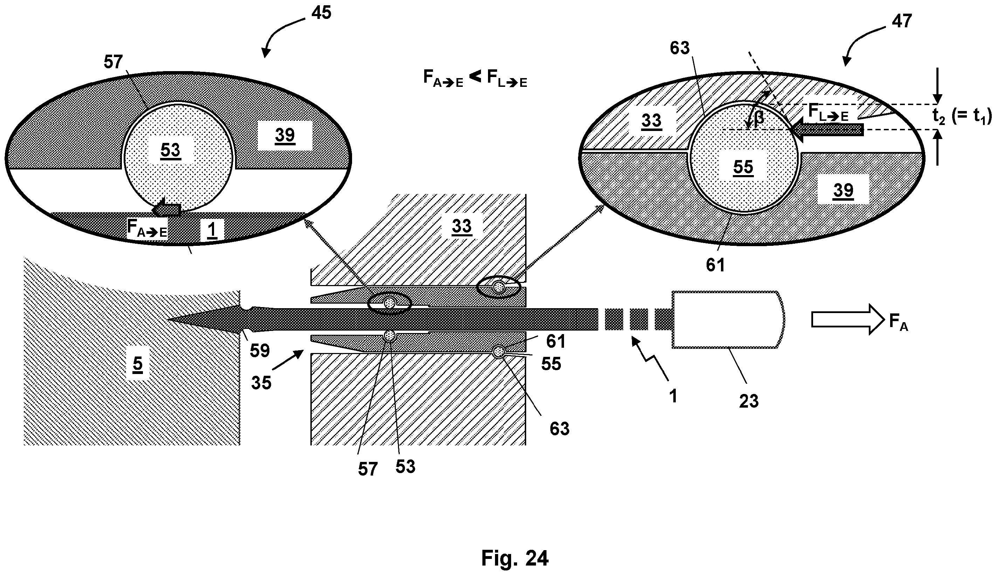

In Fig. 24 wird der Einführabschnitts 1 mit einer proximalwärtigen Herausziehkraft $F_A$ aus dem Körper 5 gezogen. Wie in Fig. 23 ist die Gleitreibungskraft, welche die Widerstandkraft $F_{A \to E}$ der ersten Schnittstelle 45 bildet, geringer als die Widerstandkraft $F_{L \to E}$ der zweiten Schnittstelle 47. Die Widerstandkraft $F_{L \to E}$ der zweiten Schnittstelle 47 wird zum einen durch die proximalseitige Eindringtiefe $t_2$ des zweiten Eingriffselements 55 in die zweite Gegennut 63 und zum anderen durch einen proximalseitigen Rampenwinkel $\beta$ der zweiten Gegennut 63 bestimmt. Der Rampenwinkel $\beta$ der zweiten Schnittstelle 47 kann dem Rampenwinkel $\varepsilon$ der ersten Schnittstelle 47 entsprechen. Damit verbleibt die Schutzhülse 39 in der maximalen Distalposition, d.h. der Sollposition, in der Aufnahme 35, wobei die Nadelspitze 9 proximalwärts in die Schutzhülse 39 gezogen wird.

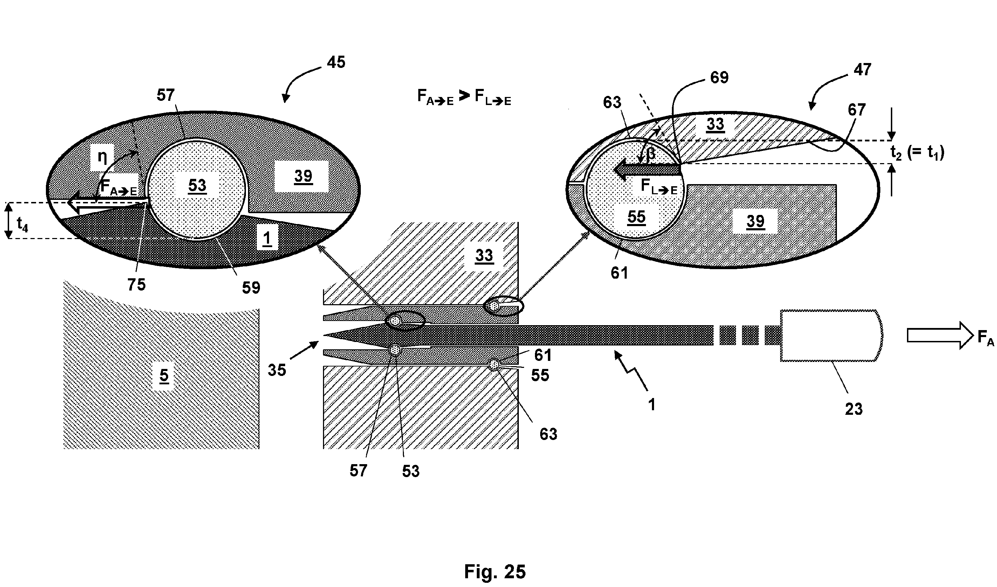

In Fig. 25 ist die erste Schnittstelle 45 wieder eingerastet, sodass sich die Schutzhülse 39 in der ersten Axialposition bezüglich des Einführabschnitts 1 befindet, in der sie die Nadelspitze 9 schützend umgreift. Durch die relativ hohe Eindringtiefe $t_4$ und den relativ steilen Rampenwinkel $\eta$, die zusammen faktisch einen proximalwärtigen Anschlag für den Einführabschnitt 1 bilden, ist nun die distalwärtige Widerstandkraft $F_{A \to E}$ der ersten Schnittstelle 45 größer als die distalwärtige Widerstandkraft $F_{L \to E}$ der zweiten Schnittstelle 47 (weil $t_4 > t_2$ und $\eta > \beta$). Dadurch drückt die zweite Gegennut 63 mit dem proximalseitigen Rampenwinkel $\beta$ das zweite Eingriffselement 55 zusammen, sodass sich dieses am proximalen Ende 69 der zweiten Gegennut 63 proximalwärts vorbeidrückt bis die zweite Schnittstelle 47 ausgerastet ist und die Schutzhülse 39 die Sollposition proximalwärts verlässt.

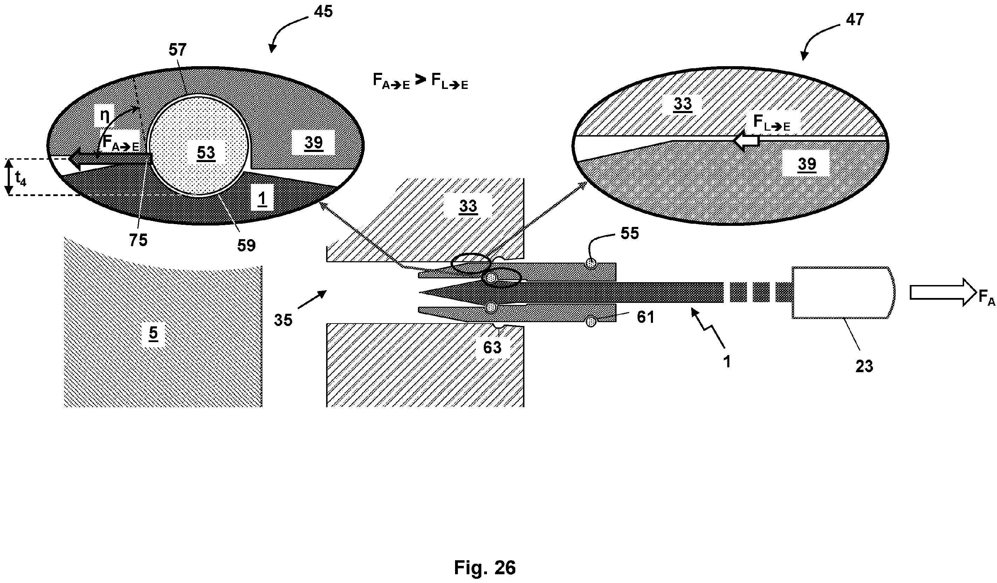

In Fig. 26 ist die zweite Schnittstelle 47 vollständig ausgerastet und die Schutzhülse 39 wird in der ersten Axialposition proximalwärts mit eingerasteter erster Schnittstelle 45 aus der Aufnahme 35 gezogen. Dabei ist die Widerstandkraft $F_{L \to E}$ der zweiten Schnittstelle 47, die hier nur noch durch eine Gleitreibungskraft erzeugt wird, wesentlich geringer als die Widerstandkraft $F_{A \to E}$ der ersten Schnittstelle 45, da die relativ hohe Eindringtiefe $t_4$ und der relativ steile Rampenwinkel $\eta$ der ersten Gegennut 59 zusammen faktisch einen distalwärtigen Anschlag für die Schutzhülse 39 bilden.

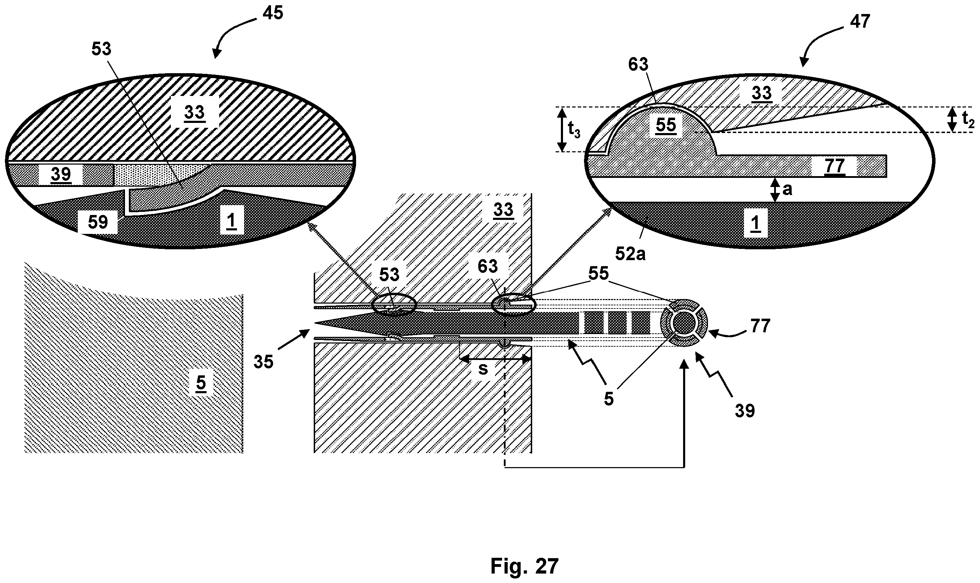

Fig. 27 zeigt eine alternative Ausführungsform, bei dem die Eingriffselemente 53, 55 integraler Bestandteil der Schutzhülse 39 sind. In anderen Ausführungsbeispielen wäre es ebenso denkbar, dass das erste Eingriffselement 53 integraler Bestandteil des Einführabschnitts 1 und/oder das zweite Eingriffselement 55 integraler Bestandteil des Positionierungselements 33 ist. Hier wird das erste Eingriffselement 53 der ersten Schnittstelle 45 durch mindestens eine auf der Innenseite der Schutzhülse 39 angeordnete Federzunge gebildet, die in eine korrespondierende Vertiefung 59 an der Außenseite des Einführabschnitts 1 eingreifen bzw. einrasten kann. Vorzugsweise wird das erste Eingriffselement 53 durch zwei oder mehr Federzungen gebildet, die umfangseitig gleichmäßig verteilt sind. Die mindestens eine Federzunge 53 ist proximalseitig angelenkt und bildet einen relativ flachen proximalseitigen Rampenwinkel, sodass die Schutzhülse 39 proximalwärts aus der ersten Axialposition bezüglich des Einführabschnitts 1 bewegt werden kann und die Federzunge 53 dabei radial nach außen nachgibt. Distalseitig bildet die Federzunge 53 einen Anschlag, der gegen eine steile distalseitige Flanke der Vertiefung 59 anschlägt, wenn die Schutzhülse 39 distalwärts in die gezeigte erste Axialposition geschoben wird. Dadurch ist die Schutzhülse 39 unverlierbar am Einführabschnitt 1 gesichert.

Das zweite Eingriffselement 55 könnte analog zum ersten Eingriffselement 53 durch eine oder mehr nach außen gerichtete Federzungen gebildet sein. In dem Ausführungsbeispiel gemäß Fig. 27 wird allerdings das zweite Eingriffselement 55 durch einen radialen Vorsprung gebildet, der sich von einer Außenseite der Schutzhülse 39 nach außen erstreckt und in eine zweite Vertiefung 63 eingreift, die hier der zweiten Gegennut 63 gemäß dem in Figuren 19 bis 26 gezeigten Ausführungsbeispiel entspricht. Hier ist die Schutzhülse 39 über eine Länge s in einem proximalen Endbereich geschlitzt, wobei Schutzhülsenendabschnitte 77 entstehen, die einen radialen Abstand a zum Einführabschnitt 1 haben und somit elastisch nach innen gebogen werden können. Dadurch kann beim Ein- und Ausrasten der zweiten Schnittstelle 47 das auf den Schutzhülsenendabschnitten 77 angeordnete Eingriffselement 55 elastisch nach innen gedrückt werden. Der radiale Abstand a ist dabei so gewählt, dass er größer oder gleich der proximalseitigen Eindringtiefe $t_2$ des zweiten Eingriffselements 55 in die zweite Vertiefung 63 ist, aber kleiner als die distalseitige Eindringtiefe $t_3$ des zweiten Eingriffselements 55 in die zweite Vertiefung 63, also $t_3 > a > t_2$ gilt. Die Eingriffselemente 53, 55 können selbst elastisch verformbar sein, aber müssen es nicht sein. Die Eingriffselemente 53, 55 können hier lediglich beweglich ausgestaltet sein.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Im Übrigen soll hierin weder der Begriff "aufweisen" zusätzliche andere Merkmale oder Verfahrensschritte ausschließen noch soll "ein" oder "eine" eine Mehrzahl ausschließen.

Bezugszeichenliste:

| | |
|---|---|
| 1 | Einführabschnitt |
| 3 | zu untersuchendes/behandelndes Gewebe |
| 5 | organischer Körper |
| 5a | haut des organischen Körpers |
| 7 | aktiv lichtemittierendes Element / LED |
| 7a | Stirnseite der LED |
| 9 | Nadelspitze |
| 9a | hülsenförmiger Abschnitt der Nadelspitze |
| 11 | Leiterelement |
| 13 | Verbindungsstelle |
| 15 | seitliche Krafteinwirkung $F_{L,S}$ |
| 17 | seitlich einwirkender externer Körper |
| 19 | Ablösekraft |
| 21 | Lichtapplikator |
| 23 | Handgriffelement |
| 25 | Anschlusskabel |
| 27 | Hand einer Bedienperson |
| 29 | seitliche Kraft $F_{L,H}$ |
| 31 | Lichtapplikatorsystem |
| 33 | Positionierelement |
| 35 | Aufnahme |
| 37 | Fixierelement |
| 39 | Schutzhülse |
| 43 | Licht der LED |
| 45 | erste Schnittstelle |
| 47 | zweite Schnittstelle |
| 49 | Gelenke |
| 51 | Behandlungstisch |
| 53 | erstes Eingriffselement |
| 55 | zweites Eingriffselement |
| 57 | erste Nut |
| 59 | erste Gegennut/Vertiefung |
| 61 | zweite Nut |
| 63 | zweite Gegennut/Vertiefung |
| 65 | Anschlag |
| 67, 68 | Rampenflächen |
| 69 | proximales Ende der zweiten Gegennut/-Vertiefung |
| 71 | distales Ende der zweiten Gegennut/Vertiefung |
| 73 | proximales Ende der ersten Gegennut/-Vertiefung |
| 75 | distales Ende der ersten Gegennut/Vertiefung |
| 77 | Schutzhülsenendabschnitte |
| $d_1$ | Länge des Einführabschnitts ohne Nadelspitze |
| $d_2$ | Länge der Nadelspitze |
| $Z_N$ | Längsachse des Einführabschnitts |
| Z | gewünschte Einstichrichtung |
| $F_A$ | manuelle Einstich- oder Herausziehkraft |
| L | axiale Länge der Aufnahme |
| $t_1$ | proximalseitige Eindringtiefe des ersten |

| | |
|---|---|
| | Eingriffelements |
| $t_2$ | proximalseitige Eindringtiefe des zweiten Eingriffelements |
| $t_3$ | distalseitige Eindringtiefe des zweiten Eingriffelements |
| $t_4$ | distalseitige Eindringtiefe des ersten Eingriffelements |
| a | radialer Abstand |
| s | Länge der Schutzhülsenendabschnitte |
| $\alpha, \beta, \gamma, \varepsilon, \eta, \delta$ | Rampenwinkel |
| $F_{A \to E}$ | Widerstandskraft der ersten Schnittstelle |
| $F_{L \to E}$ | Widerstandskraft der zweiten Schnittstelle |

**Patentansprüche**

1. Lichtapplikatorsystem (31) zur Untersuchung und/oder Behandlung von einem organischen Körper (5), wobei das Lichtapplikatorsystem (31) zumindest einen Lichtapplikator (21) und ein Positionierungselement (33) aufweist,

   wobei der Lichtapplikator (21) einen distalseitigen Einführabschnitt (1) mit zumindest einem aktiv lichtemittierenden Element (7) am distalen Ende zum Einstechen in Gewebe (3) des organischen Körpers (5) aufweist, wobei der Einführabschnitt (1) eine sich zumindest teilweise distal von dem zumindest einen aktiv lichtemittierenden Element (7) angeordnete und sich distalwärts spitz verjüngende Nadelspitze (9) aufweist,
   wobei das Positionierungselement (33) zumindest temporär in eine definierte Position und Ausrichtung relativ zum organischen Körper (5) fixierbar ist und zumindest eine Aufnahme (35) für den mindestens einen Lichtapplikator (21) aufweist, in welcher der mindestens eine Lichtapplikator (21) zumindest temporär eine definierte Ausrichtung zum organischen Körper (5) aufweist,
   **dadurch gekennzeichnet, dass** der Lichtapplikator (21) eine relativ zum Einführabschnitt (1) axial bewegliche Schutzhülse (39) aufweist, welche in einer ersten Axialposition relativ zum Einführabschnitt (1) die Nadelspitze (9) schützend umschließt und in einer durch eine Axialposition des Einführabschnitts (1) relativ zum Positionierungselement (33) bestimmten zweiten Axialposition relativ zum Einführabschnitt (1) proximalwärts von der Nadelspitze (9) zurückgeschoben ist,
   wobei die Schutzhülse (39) als Einführhülse in die mindestens eine Aufnahme (35) des Positionierungselements (33) dient.

2. Lichtapplikatorsystem (31) nach Anspruch 1, wobei die Schutzhülse (39) zumindest abschnittsweise einen Außendurchmesser aufweist, der passgenau in einen Innendurchmesser der mindestens einen Aufnahme (35) des Positionierungselements (33) passt.

3. Lichtapplikatorsystem (31) nach Anspruch 2, wobei sich der Außendurchmesser der Schutzhülse (39) am distalen Ende distalwärts verjüngt und/oder sich der Innendurchmesser der mindestens einen Aufnahme (35) des Positionierungselements (33) am proximalen Ende proximalwärts aufweitet.

4. Lichtapplikatorsystem (31) nach einem der vorhergehenden Ansprüche, wobei der Einführabschnitt (1) des Lichtapplikators (21) starr ist und in Axialrichtung ($Z_N$) eine größere Länge aufweist als die Schutzhülse (39).

5. Lichtapplikatorsystem (31) nach einem der vorhergehenden Ansprüche, wobei die Schutzhülse (39) unverlierbar am Einführabschnitt (1) des Lichtapplikators (21) gesichert ist.

6. Lichtapplikatorsystem (31) nach einem der vorhergehenden Ansprüche, wobei der Lichtapplikator (21) proximalseitig ein Handgriffelement (23) zur manuellen Positionierung des Lichtapplikators (21) aufweist.

7. Lichtapplikatorsystem (31) nach Anspruch 6, wobei die Axialposition des Einführabschnitts (1) relativ zum Positionierungselement (33) manuell durch Positionieren des Handgriffelements (23) in Axialrichtung ($Z_N$) einstellbar ist.

8. Lichtapplikatorsystem (31) nach einem der vorhergehenden Ansprüche, wobei die Schutzhülse (39) bei einem distalwärtigen Einführen des Lichtapplikators (21) in die mindestens eine Aufnahme (35) des Positionierungselements (33) so lange in der ersten Axialposition gesichert ist bis die Schutzhülse (39) in der mindestens einen Aufnahme (35) des Positionierungselements (33) eine Sollposition im Positionierungselement (33) erreicht, bei welcher der Einführabschnitt (1) distalwärts aus der Schutzhülse (39) schiebbar ist.

9. Lichtapplikatorsystem (31) nach Anspruch 8, wobei die Schutzhülse (39) bei einem proximalwärtigen Herausziehen des Lichtapplikators (21) aus der mindestens einen Aufnahme (35) des Positionierungselements (33) so lange in der Sollposition im Positionierungselement (33) gesichert ist bis die Schutzhülse (39) die erste Axialposition bezüglich des Einführabschnitts (1) einnimmt, bei welcher die Schutzhülse (39) proximalwärts aus der mindestens einen Aufnahme (35) des Positionierungselements (33) herausziehbar ist.

**10.** Lichtapplikatorsystem (31) nach einem der vorhergehenden Ansprüche, ferner mit einem elastisch verformbaren und/oder beweglichen Eingriffselement (53), wobei das Eingriffselement (53) derart zwischen dem Einführabschnitt (1) und der Schutzhülse (39) angeordnet ist, dass es bei Überwindung einer Axialkraft so weit elastisch nachgibt oder sich bewegt, dass die Schutzhülse (39) die erste Axialposition distalwärts erreichen und/oder proximalwärts verlassen kann.

**11.** Lichtapplikatorsystem (31) nach Anspruch 10, wobei das Eingriffselement (53) ein erstes von mindestens zwei elastisch verformbaren und/oder beweglichen Eingriffselementen (53, 55) mit einem zweiten Eingriffselement (55) ist und die Axialkraft eine erste Axialkraft von mindestens zwei Axialkräften mit einer zweiten Axialkraft ist, wobei das zweite Eingriffselement (55) derart zwischen der Schutzhülse (39) und der mindestens einen Aufnahme (35) des Positionierungselements (33) angeordnet ist, dass es bei Überwindung der zweiten Axialkraft so weit elastisch nachgibt oder sich bewegt, dass die Schutzhülse (39) die Sollposition im Positionierungselement (33) distalwärts erreichen und/oder proximalwärts verlassen kann.

**12.** Lichtapplikatorsystem (31) nach Anspruch 10 oder 11, wobei das erste Eingriffselement (53) Teil der Schutzhülse (39) und/oder des Einführabschnitts (1) ist.

**13.** Lichtapplikatorsystem (1) nach einem der Ansprüche 10 bis 12, wobei das zweite Eingriffselement (55) Teil der Schutzhülse (39) und/oder des Positionierungselements (33) ist.

**14.** Lichtapplikatorsystem (1) nach einem der Ansprüche 10 bis 13, wobei distalwärts die erste Axialkraft größer ist als die zweite Axialkraft und proximalwärts die zweite Axialkraft größer ist als die erste Axialkraft.

**Claims**

**1.** Light applicator system (31) for the examination and/or treatment of an organic body (5), wherein the light applicator system (31) has at least one light applicator (21) and a positioning element (33),

> wherein the light applicator (21) has a distal-side insertion section (1) with at least one active light-emitting element (7) at the distal end for the penetration of tissue (3) of the organic body (5), wherein the insertion section (1) has a needle tip (9), which, at least partially, is arranged distally from the at least one active light-emitting element (7), and tapers to a point in the distal direction,
wherein the positioning element (33) can be fixed at least temporarily in a defined position and orientation relative to the organic body (5) and has at least one repository (35) for the at least one light applicator (21), in which the at least one light applicator (21) has, at least temporarily, a defined orientation relative to the organic body (5),
**characterised in that** the light applicator (21) has a protective sleeve (39) which is axially movable relative to the insertion section (1); it encloses the needle tip (9) in a protective manner in a first axial position relative to the insertion section (1), and in a second axial position, determined by an axial position of the insertion section (1) relative to the positioning element (33), is pushed back in a proximal direction from the needle tip (9) relative to the insertion section (1),
wherein the protective sleeve (39) serves as an insertion sleeve into the at least one repository (35) of the positioning element (33).

**2.** Light applicator system (31) in accordance with Claim 1, wherein the protective sleeve (39) has an outer diameter, which, at least in some sections, fits precisely into an inner diameter of the at least one repository (35) of the positioning element (33).

**3.** Light applicator system (31) in accordance with Claim 2, wherein at the distal end the outer diameter of the protective sleeve (39) tapers in the distal direction, and/or at the proximal end the inner diameter of the at least one repository (35) of the positioning element (33) widens in the proximal direction.

**4.** Light applicator system (31) in accordance with one of the preceding claims, wherein the insertion section (1) of the light applicator (21) is rigid, and in the axial direction ($Z_N$) has a greater length than the protective sleeve (39).

**5.** Light applicator system (31) in accordance with one of the preceding claims, wherein the protective sleeve (39) is captively secured to the insertion section (1) of the light applicator (21).

**6.** Light applicator system (31) in accordance with one of the preceding claims, wherein on the proximal side the light applicator (21) has a handgrip element (23) for the manual positioning of the light applicator (21).

**7.** Light applicator system (31) in accordance with Claim 6, wherein the axial position of the insertion section (1) relative to the positioning element (33) can be adjusted manually by positioning of the handgrip element (23) in the axial direction ($Z_N$).

8. Light applicator system (31) in accordance with one of the preceding claims, wherein when the light applicator (21) is inserted in the distal direction into the at least one repository (35) of the positioning element (33), the protective sleeve (39) is secured in the first axial position, until the protective sleeve (39) in the at least one repository (35) of the positioning element (33) achieves a desired position in the positioning element (33), in which the insertion section (1) can pushed in the distal direction out of the protective sleeve (39).

9. Light applicator system (31) in accordance with Claim 8, wherein when the light applicator (21) is pulled in the proximal direction out of the at least one repository (35) of the positioning element (33), the protective sleeve (39) is secured in the desired position in the positioning element (33) until the protective sleeve (39) assumes the first axial position with respect to the insertion section (1), in which the protective sleeve (39) can be pulled in the proximal direction out of the at least one repository (35) of the positioning element (33).

10. Light applicator system (31) in accordance with one of the preceding claims, further comprising an elastically deformable, and/or movable, engagement element (53), wherein the engagement element (53) is arranged between the insertion section (1) and the protective sleeve (39), such that, when an axial force is overcome, it yields elastically or moves to an extent such that the protective sleeve (39) can reach the first axial position in the distal direction, and/or leave it in the proximal direction.

11. Light applicator system (31) in accordance with Claim 10, wherein the engagement element (53) is a first of at least two elastically deformable and/or movable engagement elements (53, 55), with a second engagement element (55), and the axial force is a first axial force of at least two axial forces, with a second axial force, wherein the second engagement element (55) is arranged between the protective sleeve (39) and the at least one repository (35) of the positioning element (33) such that, when the second axial force is overcome, it yields elastically, or moves to an extent such that the protective sleeve (39) can reach the desired position in the positioning element (33) in the distal direction, and/or leave it in the proximal direction.

12. Light applicator system (31) in accordance with Claim 10 or 11, wherein the first engagement element (53) is part of the protective sleeve (39) and/or the insertion section (1).

13. Light applicator system (1) in accordance with one of the Claims 10 to 12, wherein the second engagement element (55) is part of the protective sleeve (39) and/or the positioning element (33).

14. Light applicator system (1) in accordance with one of the Claims 10 to 13, wherein in the distal direction, the first axial force is greater than the second axial force, and in the proximal direction, the second axial force is greater than the first axial force.

## Revendications

1. Système d'applicateur de lumière (31) pour examiner et/ou traiter un corps organique (5), dans lequel le système d'applicateur de lumière (31) présente au moins un applicateur de lumière (21) et un élément de positionnement (33),

   dans lequel l'applicateur de lumière (21) présente un tronçon d'introduction côté distal (1) comprenant au moins un élément d'émission de lumière actif (7) sur l'extrémité distale pour piquer dans le tissu (3) du corps organique (5), dans lequel le tronçon d'introduction (1) présente une pointe d'aiguille (9) disposée au moins partiellement de manière distale de l'au moins un élément d'émission de lumière actif (7) et se réduisant en pointe vers le côté distal, dans lequel l'élément de positionnement (33) peut être fixé au moins temporairement dans une position et une orientation définies par rapport au corps organique (5) et présente au moins une réception (35) pour l'au moins un applicateur de lumière (21), dans lequel l'au moins un applicateur de lumière (21) présente au moins temporairement une orientation définie par rapport au corps organique (5),
   **caractérisé en ce que** l'applicateur de lumière (21) présente une douille protectrice (39) mobile axialement par rapport au tronçon d'introduction (1), laquelle entoure la pointe d'aiguille (9) de manière protectrice par rapport au tronçon d'introduction (1) dans une première position axiale et est retirée dans une deuxième position axiale vers la direction proximale de la pointe d'aiguille (9) par rapport au tronçon d'introduction (1) définie par une position axiale du tronçon d'introduction (1) par rapport à l'élément de positionnement (33),
   dans lequel la douille protectrice (39) sert de douille d'introduction dans l'au moins une réception (35) de l'élément de positionnement (33).

2. Système d'applicateur de lumière (31) selon la revendication 1, dans lequel la douille protectrice (39) présente un diamètre extérieur au moins par tronçons qui passe précisément dans un diamètre intérieur de l'au moins une réception (35) de l'élément de positionnement (33).

3.  Système d'applicateur de lumière (31) selon la revendication 2, dans lequel le diamètre extérieur de la douille protectrice (39) se rétrécit vers le côté distal sur l'extrémité distale et/ou le diamètre intérieur de l'au moins une réception (35) de l'élément de positionnement (33) s'élargit vers le côté proximal sur l'extrémité proximale.

4.  Système d'applicateur de lumière (31) selon l'une des revendications précédentes, dans lequel le tronçon d'introduction (1) de l'applicateur de lumière (21) est fixe et présente dans la direction axiale ($Z_N$) une longueur plus grande que la douille protectrice (39).

5.  Système d'applicateur de lumière (31) selon l'une des revendications précédentes, dans lequel la douille protectrice (39) est fixée de manière imperdable sur le tronçon d'introduction (1) de l'applicateur de lumière (21).

6.  Système d'applicateur de lumière (31) selon l'une des revendications précédentes, dans lequel l'applicateur de lumière (21) présente, côté proximal, un élément de poignée (23) pour le positionnement manuel de l'applicateur de lumière (21).

7.  Système d'applicateur de lumière (31) selon la revendication 6, dans lequel la position axiale du tronçon d'introduction (1) par rapport à l'élément de positionnement (33) est réglable manuellement par le positionnement de l'élément de poignée (23) dans la direction axiale ($Z_N$).

8.  Système d'applicateur de lumière (31) selon l'une des revendications précédentes, dans lequel la douille protectrice (39), lors d'une introduction de l'applicateur de lumière (21) vers le côté distal dans l'au moins une réception (35) de l'élément de positionnement (33), est fixée dans la première position axiale jusqu'à ce que la douille protectrice (39) dans l'au moins une réception (35) de l'élément de positionnement (33) atteigne une position nominale dans l'élément de positionnement (33), dans laquelle le tronçon d'introduction (1) peut être poussé hors de la douille protectrice (39) vers le côté distal.

9.  Système d'applicateur de lumière (31) selon la revendication 8, dans lequel la douille protectrice (39), lors d'une sortie vers le côté proximal de l'applicateur de lumière (21) hors de l'au moins une réception (35) de l'élément de positionnement (33), est fixée dans la position nominale dans l'élément de positionnement (33) jusqu'à ce que la douille protectrice (39) adopte la première position axiale par rapport au tronçon d'introduction (1), dans laquelle la douille protectrice (39) peut être sortie de l'au moins une réception (35) de l'élément de positionnement (33) dans le sens proximal.

10. Système d'applicateur de lumière (31) selon l'une des revendications précédentes, comprenant en outre un élément d'intervention (53) déformable élastiquement et/ou mobile, dans lequel l'élément d'intervention (53) est ainsi disposé entre le tronçon d'introduction (1) et la douille protectrice (39) qu'il se relâche ou se déplace élastiquement si loin lorsqu'une force axiale est dépassée, que la douille protectrice (39) peut atteindre la première position axiale vers le côté distal et/ou quitter la première position axiale vers le côté proximal.

11. Système d'applicateur de lumière (31) selon la revendication 10, dans lequel l'élément d'intervention (53) est un premier d'au moins deux éléments d'intervention (53, 55) déformables élastiquement et/ou mobiles avec un second élément d'intervention (55), et la force axiale est une première force axiale d'au moins deux forces axiales avec une seconde force axiale, dans lequel le second élément d'intervention (55) est ainsi disposé entre la douille protectrice (39) et l'au moins une réception (35) de l'élément de positionnement (33) que lorsque la seconde force axiale est dépassée, il se relâche ou se déplace élastiquement si loin que la douille protectrice (39) peut atteindre la position nominale dans l'élément de positionnement (33) vers le côté distal et/ou quitter la position nominale vers le côté proximal.

12. Système d'applicateur de lumière (31) selon la revendication 10 ou 11, dans lequel le premier élément d'intervention (53) fait partie de la douille protectrice (39) et/ou du tronçon d'introduction (1).

13. Système d'applicateur de lumière (1) selon l'une des revendications 10 à 12, dans lequel le second élément d'intervention (55) fait partie de la douille protectrice (39) et/ou de l'élément de positionnement (33).

14. Système d'applicateur de lumière (1) selon l'une des revendications 10 à 13, dans lequel vers le côté distal, la première force axiale est plus grande que la seconde force axiale et côté proximal, la seconde force axiale est plus grande que la première force axiale.

Fig. 1a

Fig. 1b

EP 4 337 310 B1

Fig. 2

Fig. 3b

Fig. 3a

Fig. 4

**Fig. 5**

Fig. 6

Fig. 7

Fig. 8

**Fig. 9**

Fig. 10

Fig. 11

$$F_{A \to E} > F_{L \to E}$$

Fig. 12

$F_A$

23

$F_{A \to E} < F_{L \to E}$

1

39

33

$F_{A \to E}$

$F_{L \to E}$

47

45

5

Fig. 13

**Fig. 14**

$$F_{A \to E} < F_{L \to E}$$

$F_{L \to E}$

$F_{A \to E}$

$F_A$

5

33

39

1

23

45 47

EP 4 337 310 B1

30

$F_{A \to E} < F_{L \to E}$

$F_{L \to E}$

$F_{A \to E}$

2

33

45   47

39

1

23

$F_A$

**Fig. 15**

EP 4 337 310 B1

Fig. 16

Fig. 17

**Fig. 18**

EP 4 337 310 B1

Fig. 19

Fig. 20

**Fig. 21**

EP 4 337 310 B1

$F_{A \to E} < F_{L \to E}$

Fig. 22

EP 4 337 310 B1

Fig. 23

Fig. 24

Fig. 25

**Fig. 26**

**Fig. 27**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6048359 A
- DE 102019129556 A1
- DE 102019212197 A1